# EUROPÄISCHE PATENTSCHRIFT

(11) **EP 0 977 867 B1**
(45) Veröffentlichungstag und Bekanntmachung des Hinweises auf die Patenterteilung: **07.03.2007**
(21) Anmeldenummer: 98940118.7
(22) Anmeldetag: 02.07.1998
(51) Int. Cl.: C12N 15/54, C12N 9/10, C12N 5/10, C12N 1/21, C12P 17/18

(54) **VERFAHREN ZUR HERSTELLUNG VON BIOTIN**
METHOD FOR PRODUCING BIOTIN
PROCEDE DE PRODUCTION DE BIOTINE

(30) Priorität: 22.07.1997 DE 19731274
(43) Veröffentlichungstag der Anmeldung: 09.02.2000
(73) Patentinhaber: BASF Aktiengesellschaft, 67056 Ludwigshafen (DE)
(72) Erfinder: SCHRÖDER, Hartwig, D-69226 Nussloch (DE); HAUER, Bernhard, D-67136 Fussgönheim (DE)
(86) Internationale Anmeldenummer: PCT/EP1998/004097
(87) Internationale Veröffentlichungsnummer: WO 1999/005285

(56) Entgegenhaltungen:
- EP-A- 0 635 572
- EP-A- 0 806 479
- ZHENG L ET AL: "CATALYTIC FORMATION OF A NITROGENASE IRON-SULFUR CLUSTER" JOURNAL OF BIOLOGICAL CHEMISTRY, Bd. 269, Nr. 29, 22. Juli 1994, Seiten 18723-18726, XP002034651
- SANYAL I ET AL: "BIOTIN SYNTHASE: PURIFICATION, CHARACTERIZATION AS A 2FE-2SCLUSTER PROTEIN, AND IN VITRO ACTIVITY OF THE ESCHERICHIA COLI BIOB GENE PRODUCT" BIOCHEMISTRY, Bd. 33, Nr. 12, 1994, Seiten 3625-3631, XP002034650
- IFUKU O ET AL: "FLAVODOXIN IS REQUIRED FOR CONVERSION OF DETHIOBIOTIN TO BIOTIN IN ESCHERICHIA COLI" EUROPEAN JOURNAL OF BIOCHEMISTRY, Bd. 224, Nr. 1, August 1994, Seiten 173-178, XP002034653
- ZHENG L ET AL: "CYSTEINE DESULFURASE ACTIVITY INDICATES A ROLE FOR NIFS IN METALLOCLUSTER BIOSYNTHESIS" PROCEEDINGS OF THE NATIONAL ACADEMY OF SCIENCES OF USA, Bd. 90, April 1993, Seiten 2754-2758, XP002034652 in der Anmeldung erwähnt

### SEQUENZPROTOKOLL

(1) ALGEMEINE INFORMATION:
   (i) ANMELDER:
      (A) NAME: BASF Aktiengesellschaft
      (B) STRASSE: Carl Bosch Strasse
      (C) ORT: Ludwigshafen
      (D) BUNDESLAND: Rheinland-Pfalz
      (E) LAND: Germany
      (F) POSTLEITZAHL: D-67056
   (ii) ANMELDETITEL: Verfahren zur Herstellung von Biotin
   (iii) ANZAHL DER SEQUENZEN: 12
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.25 (EPA)
(2) INFORMATION ZU SEQ ID NO: 1:
   (i) SEQUENZ CHARAKTERISTIKA:
      (A) LÄNGE: 1217 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
      (B) STAMM: W3110
   (ix) MERKMALE:
      (A) NAME/SCHLÜSSEL: 5'UTR
      (B) LAGE: 1..11
   (ix) MERKMALE:
      (A) NAME/SCHLÜSSEL: CDS
      (B) LAGE: 12..1217
   (xi) SEQUENZBESCHREIBUNG: SEQ ID NO: 1:
(2) INFORMATION ZU SEQ ID NO: 2:
   (i) SEQUENZ CHARAKTERISTIKA:
      (A) LÄNGE: 401 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Asn Val Phe Asn Pro Ala Gln Phe Arg Ala Gln Phe Pro Ala Leu
    1               5                  10                  15
   Gln Asp Ala Gly Val Tyr Leu Asp Ser Ala Ala Thr Ala Leu Lys Pro
               20                  25                  30
   Glu Ala Val Val Glu Ala Thr Gln Gln Phe Tyr Ser Leu Ser Ala Gly
           35                  40                  45
   Asn Val His Arg Ser Gln Phe Ala Glu Ala Gln Arg Leu Thr Ala Arg
       50                  55                  60
   Tyr Glu Ala Ala Arg Glu Lys Val Ala Gln Leu Leu Asn Ala Pro Asp
   65                  70                  75                  80
   Asp Lys Thr Ile Val Trp Thr Arg Gly Thr Thr Glu Ser Ile Asn Met
                   85                  90                  95
   Val Ala Gln Cys Tyr Ala Arg Pro Arg Leu Gln Pro Gly Asp Glu Ile
               100                 105                 110
   Ile Val Ser Val Ala Glu His His Ala Asn Leu Val Pro Trp Leu Met
           115                 120                 125
   Val Ala Gln Gln Thr Gly Ala Lys Val Val Lys Leu Pro Leu Asn Ala
       130                 135                 140
   Gln Arg Leu Pro Asp Val Asp Leu Leu Pro Glu Leu Ile Thr Pro Arg
   145                 150                 155                 160
   Ser Arg Ile Leu Ala Leu Gly Gln Met Ser Asn Val Thr Gly Gly Cys
                   165                 170                 175
   Pro Asp Leu Ala Arg Ala Ile Thr Phe Ala His Ser Ala Gly Met Val
               180                 185                 190
   Val Met Val Asp Gly Ala Gln Gly Ala Val His Phe Pro Ala Asp Val
           195                 200                 205
   Gln Gln Leu Asp Ile Asp Phe Tyr Ala Phe Ser Gly His Lys Leu Tyr
       210                 215                 220
   Gly Pro Thr Gly Ile Gly Val Leu Tyr Gly Lys Ser Glu Leu Leu Glu
   225                 230                 235                 240
   Ala Met Ser Pro Trp Leu Gly Gly Gly Lys Met Val His Glu Val Ser
                   245                 250                 255
   Phe Asp Gly Phe Thr Thr Gln Ser Ala Pro Trp Lys Leu Glu Ala Gly
               260                 265                     270
   Thr Pro Asn Val Ala Gly Val Ile Gly Leu Ser Ala Ala Leu Glu Trp
           275                 280                 285
   Leu Ala Asp Tyr Asp Ile Asn Gln Ala Glu Ser Trp Ser Arg Ser Leu
       290                 295                 300
   Ala Thr Leu Ala Glu Asp Ala Leu Ala Lys Arg Pro Gly Phe Arg Ser
   305                 310                 315                 320
   Phe Arg Cys Gln Asp Ser Ser Leu Leu Ala Phe Asp Phe Ala Gly Val
                   325                 330                 335
   His His Ser Asp Met Val Thr Leu Leu Ala Glu Tyr Gly Ile Ala Leu
               340                 345                 350
   Arg Ala Gly Gln His Cys Ala Gln Pro Leu Leu Ala Glu Leu Gly Val
           355                 360                 365
   Thr Gly Thr Leu Arg Ala Ser Phe Ala Pro Tyr Asn Thr Lys Ser Asp
       370                 375                 380
   Val Asp Ala Leu Val Asn Ala Val Asp Arg Ala Leu Glu Leu Leu Val
   385                 390                 395                 400
   Asp
(2) INFORMATION ZU SEQ ID NO: 3:
   (i) SEQUENZ CHARAKTERISTIKA:
      (A) LÄNGE: 1233 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Escherichia coli
      (B) STAMM: W3110
   (ix) MERKMALE:
      (A) NAME/SCHLÜSSEL: CDS
      (B) LAGE: 19..1233
   (ix) MERKMALE:
      (A) NAME/SCHLÜSSEL: 5'UTR
      (B) LAGE: 1..18
   (xi) SEQUENZBESCHREIBUNG: SEQ ID NO: 3:
(2) INFORMATION ZU SEQ ID NO: 4:
   (i) SEQUENZ CHARAKTERISTIKA:
      (A) LÄNGE: 404 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
   Met Lys Leu Pro Ile Tyr Leu Asp Tyr Ser Ala Thr Thr Pro Val Asp
     1               5                  10                  15
   Pro Arg Val Ala Glu Lys Met Met Gln Phe Met Thr Met Asp Gly Thr
               20                  25                  30
   Phe Gly Asn Pro Ala Ser Arg Ser His Arg Phe Gly Trp Gln Ala Glu
           35                  40                  45
   Glu Ala Val Asp Ile Ala Arg Asn Gln Ile Ala Asp Leu Val Gly Ala
       50                  55                  60
   Asp Pro Arg Glu Ile Val Phe Thr Ser Gly Ala Thr Glu Ser Asp Asn
   65                  70                  75                  80
   Leu Ala Ile Lys Gly Ala Ala Asn Phe Tyr Gln Lys Lys Gly Lys His
                   85                  90                  95
   Ile Ile Thr Ser Lys Thr Glu His Lys Ala Val Leu Asp Thr Cys Arg
               100                 105                 110
   Gln Leu Glu Arg Glu Gly Phe Glu Val Thr Tyr Leu Ala Pro Gln Arg
           115                 120                 125
   Asn Gly Ile Ile Asp Leu Lys Glu Leu Glu Ala Ala Met Arg Asp Asp
       130                 135                 140
   Thr Ile Leu Val Ser Ile Met His Val Asn Asn Glu Ile Gly Val Val
   145                 150                 155                 160
   Gln Asp Ile Ala Ala Ile Gly Glu Met Cys Arg Ala Arg Gly Ile Ile
                   165                 170                 175
   Tyr His Val Asp Ala Thr Gln Ser Val Gly Lys Leu Pro Ile Asp Leu
               180                 185                 190
   Ser Gln Leu Lys Val Asp Leu Met Ser Phe Ser Gly His Lys Ile Tyr
           195                 200                 205
   Gly Pro Lys Gly Ile Gly Ala Leu Tyr Val Arg Arg Lys Pro Arg Val
       210                 215                 220
   Arg Ile Glu Ala Gln Met His Gly Gly Gly His Glu Arg Gly Met Arg
   225                 230                 235                 240
   Ser Gly Thr Leu Pro Val His Gln Ile Val Gly Met Gly Glu Ala Tyr
                   245                 250                 255
   Arg Ile Ala Lys Glu Glu Met Ala Thr Glu Met Glu Arg Leu Arg Gly
               260                 265                 270
   Leu Arg Asn Arg Leu Trp Asn Gly Ile Lys Asp Ile Glu Glu Val Tyr
           275                 280                 285
   Leu Asn Gly Asp Leu Glu His Gly Ala Pro Asn Ile Leu Asn Val Ser
       290                 295                 300
   Phe Asn Tyr Val Glu Gly Glu Ser Leu Ile Met Ala Leu Lys Asp Leu
   305                 310                 315                 320
   Ala Val Ser Ser Gly Ser Ala Cys Thr Ser Ala Ser Leu Glu Pro Ser
                   325                 330                 335
   Tyr Val Leu Arg Ala Leu Gly Leu Asn Asp Glu Leu Ala His Ser Ser
               340                 345                 350
   Ile Arg Phe Ser Leu Gly Arg Phe Thr Thr Glu Glu Glu Ile Asp Tyr
           355                 360                 365
   Thr Ile Glu Leu Val Arg Lys Ser Ile Gly Arg Leu Arg Asp Leu Ser
       370                 375                 380
   Pro Leu Trp Glu Met Tyr Lys Gln Gly Val Asp Leu Asn Ser Ile Glu
   385                 390                 395                 400
   Trp Ala His His
(2) INFORMATION ZU SEQ ID NO: 5:
   (i) SEQUENZ CHARAKTERISTIKA:
      (A) LÄNGE: 3794 Basenpaare
      (B) ART: Nukleinsäure
      (C) STRANGFORM: Einzel
      (D) TOPOLOGIE: ringförmig
   (ii) ART DES MOLEKÜLS: DNS (genomisch)
   (iii) HYPOTHETISCH: NEIN
   (iii) ANTISENSE: NEIN
   (vii) UNMITTELBARE HERKUNFT:
      (B) CLON: pHS1bioS1
   (ix) MERKMALE:
      (A) NAME/SCHLÜSSEL: CDS
      (B) LAGE: 601..1806
   (xi) SEQUENZBESCHREIBUNG: SEQ ID NO: 5:
   GACGTCTGTG TGGAATTGTG AGCGGATAAC AATTTCACAC AGGGCCCTCG GACACCGAGG      60
   AGAATGTCAA GAGGCGAACA CACAACGTCT TGGAGCGCCA GAGGAGGAAC GAGCTAAAAC     120
   GGAGCTTTTT TGCCCTGCGT GACCAGATCC CGGAGTTGGA AAACAATGAA AAGGCCCCCA     180
   AGGTAGTTAT CCTTAAAAAA GCCACAGCAT ACATCCTGTC CGTCCAAGCA GAGGAGCAAA     240
   AGCTCATTTC TGAAGAGGAC TTGTTGCGGA AACGACGAGA ACAGTTGAAA CACAAACTTG     300
   AACAGCTACG GAACTCTTGT GCGTAAGGAA AAGTAAGGAA AACGATTCCT TCTAACAGAA     360
   ATGTCCTGAG CAATCACCTA TGAACTGTCG ACTCGAGATA GCATTTTTAT CCATAAGATT     420
   AGCCGATCCT AAGGTTTACA ATTGTGAGCG CTCACAATTA TGATAGATTC AATTGTGAGC     480
   GGATAACAAT TTCACACACG CTAGCGGTAC CGGGCCCCCC CTCGAGGTCG ACGGTATCGA     540
   TAAGCTTGAT ATCGAATTCC TGCAGCCCGG GGGATCCCAT GGTACGCGTC GAGGAGTACC     600
   ATG AAC GTT TTT AAT CCC GCG CAG TTT CGC GCC CAG TTT CCC GCA CTA      648
   Met Asn Val Phe Asn Pro Ala Gln Phe Arg Ala Gln Phe Pro Ala Leu
     1               5                  10                  15
   CAG GAT GCG GGC GTC TAT CTC GAC AGC GCC GCG ACC GCG CTT AAA CCT      696
   Gln Asp Ala Gly Val Tyr Leu Asp Ser Ala Ala Thr Ala Leu Lys Pro
                20                  25                  30
   GAA GCC GTG GTT GAA GCC ACC CAA CAG TTT TAC AGT CTG AGC GCC GGA      744
   Glu Ala Val Val Glu Ala Thr Gln Gln Phe Tyr Ser Leu Ser Ala Gly
            35                  40                  45
   AAC GTC CAT CGC AGC CAG TTT GCC GAA GCC CAA CGC CTG ACC GCG CGT      792
   Asn Val His Arg Ser Gln Phe Ala Glu Ala Gln Arg Leu Thr Ala Arg
        50                  55                  60
   TAT GAA GCT GCA CGA GAG AAA GTG GCG CAA TTA CTG AAT GCA CCG GAT      840
   Tyr Glu Ala Ala Arg Glu Lys Val Ala Gln Leu Leu Asn Ala Pro Asp
    65                  70                  75                  80
   GAT AAA ACT ATC GTC TGG ACG CGC GGC ACC ACT GAA TCC ATC AAC ATG      888
   Asp Lys Thr Ile Val Trp Thr Arg Gly Thr Thr Glu Ser Ile Asn Met
                    85                  90                  95
   GTG GCA CAA TGC TAT GCG CGT CCG CGT CTG CAA CCG GGC GAT GAG ATT      936
   Val Ala Gln Cys Tyr Ala Arg Pro Arg Leu Gln Pro Gly Asp Glu Ile
               100                 105                 110
   ATT GTC AGC GTG GCA GAA CAC CAC GCC AAC CTC GTC CCC TGG CTG ATG      984
   Ile Val Ser Val Ala Glu His His Ala Asn Leu Val Pro Trp Leu Met
           115                 120                 125
   GTC GCC CAA CAA ACT GGA GCC AAA GTG GTG AAA TTG CCG CTT AAT GCG     1032
   Val Ala Gln Gln Thr Gly Ala Lys Val Val Lys Leu Pro Leu Asn Ala
       130                 135                 140
   CAG CGA CTG CCG GAT GTC GAT TTG TTG CCA GAA CTG ATT ACT CCC CGT     1080
   Gln Arg Leu Pro Asp Val Asp Leu Leu Pro Glu Leu Ile Thr Pro Arg
   145                 150                 155                 160
   AGT CGG ATT CTG GCG TTG GGT CAG ATG TCG AAC GTT ACT GGC GGT TGC     1128
   Ser Arg Ile Leu Ala Leu Gly Gln Met Ser Asn Val Thr Gly Gly Cys
                   165                 170                 175
   CCG GAT CTG GCG CGA GCG ATT ACC TTT GCT CAT TCA GCC GGG ATG GTG     1176
   Pro Asp Leu Ala Arg Ala Ile Thr Phe Ala His Ser Ala Gly Met Val
               180                 185                 190
   GTG ATG GTT GAT GGT GCT CAG GGG GCA GTG CAT TTC CCC GCG GAT GTT     1224
   Val Met Val Asp Gly Ala Gln Gly Ala Val His Phe Pro Ala Asp Val
           195                 200                 205
   CAG CAA CTG GAT ATT GAT TTC TAT GCT TTT TCA GGT CAC AAA CTG TAT     1272
   Gln Gln Leu Asp Ile Asp Phe Tyr Ala Phe Ser Gly His Lys Leu Tyr
       210                 215                 220
   GGC CCG ACA GGT ATC GGC GTG CTG TAT GGT AAA TCA GAA CTG CTG GAG     1320
   Gly Pro Thr Gly Ile Gly Val Leu Tyr Gly Lys Ser Glu Leu Leu Glu
   225                 230                 235                 240
   GCG ATG TCG CCC TGG CTG GGC GGC GGC AAA ATG GTT CAC GAA GTG AGT     1368
   Ala Met Ser Pro Trp Leu Gly Gly Gly Lys Met Val His Glu Val Ser
                   245                 250                 255
   TTT GAC GGC TTC ACG ACT CAA TCT GCG CCG TGG AAA CTG GAA GCT GGA     1416
   Phe Asp Gly Phe Thr Thr Gln Ser Ala Pro Trp Lys Leu Glu Ala Gly
               260                 265                 270
   ACG CCA AAT GTC GCT GGT GTC ATA GGA TTA AGC GCG GCG CTG GAA TGG     1464
   Thr Pro Asn Val Ala Gly Val Ile Gly Leu Ser Ala Ala Leu Glu Trp
           275                 280                 285
   CTG GCA GAT TAC GAT ATC AAC CAG GCC GAA AGC TGG AGC CGT AGC TTA     1512
   Leu Ala Asp Tyr Asp Ile Asn Gln Ala Glu Ser Trp Ser Arg Ser Leu
       290                 295                 300
   GCA ACG CTG GCG GAA GAT GCG CTG GCG AAA CGT CCC GGC TTT CGT TCA      1560
   Ala Thr Leu Ala Glu Asp Ala Leu Ala Lys Arg Pro Gly Phe Arg Ser
   305                 310                 315                 320
   TTC CGC TGC CAG GAT TCC AGC CTG CTG GCC TTT GAT TTT GCT GGC GTT      1608
   Phe Arg Cys Gln Asp Ser Ser Leu Leu Ala Phe Asp Phe Ala Gly Val
                   325                 330                 335
   CAT CAT AGC GAT ATG GTG ACG CTG CTG GCG GAG TAC GGT ATT GCC CTG      1656
   His His Ser Asp Met Val Thr Leu Leu Ala Glu Tyr Gly Ile Ala Leu
               340                 345                 350
   CGG GCC GGG CAG CAT TGC GCT CAG CCG CTA CTG GCA GAA TTA GGC GTA      1704
   Arg Ala Gly Gln His Cys Ala Gln Pro Leu Leu Ala Glu Leu Gly Val
           355                 360                 365
   ACC GGC ACA CTG CGC GCC TCT TTT GCG CCA TAT AAT ACA AAG AGT GAT      1752
   Thr Gly Thr Leu Arg Ala Ser Phe Ala Pro Tyr Asn Thr Lys Ser Asp
       370                 375                 380
   GTG GAT GCG CTG GTG AAT GCC GTT GAC CGC GCG CTG GAA TTA TTG GTG      1800
   Val Asp Ala Leu Val Asn Ala Val Asp Arg Ala Leu Glu Leu Leu Val
   385                 390                 395                 400
   GAT TAAACGCGTG CTAGAGGCAT CAAATAAAAC GAAAGGCTCA GTCGAAAGAC          1853
   Asp
   TGGGCCTTTC GTTTTATCTG TTGTTTGTCG GTGAACGCTC TCCTGAGTAG GACAAATCCG    1913
   CCGCCCTAGA CCTAGGGGAT ATATTCCGCT TCCTCGCTCA CTGACTCGCT ACGCTCGGTC    1973
   GTTCGACTGC GGCGAGCGGA AATGGCTTAC GAACGGGGCG GAGATTTCCT GGAAGATGCC    2033
   AGGAAGATAC TTAACAGGGA AGTGAGAGGG CCGCGGCAAA GCCGTTTTTC CATAGGCTCC    2093
   GCCCCCCTGA CAAGCATCAC GAAATCTGAC GCTCAAATCA GTGGTGGCGA AACCCGACAG    2153
   GACTATAAAG ATACCAGGCG TTTCCCCCTG GCGGCTCCCT CGTGCGCTCT CCTGTTCCTG    2213
   CCTTTCGGTT TACCGGTGTC ATTCCGCTGT TATGGCCGCG TTTGTCTCAT TCCACGCCTG    2273
   ACACTCAGTT CCGGGTAGGC AGTTCGCTCC AAGCTGGACT GTATGCACGA ACCCCCCGTT    2333
   CAGTCCGACC GCTGCGCCTT ATCCGGTAAC TATCGTCTTG AGTCCAACCC GGAAAGACAT    2393
   GCAAAAGCAC CACTGGCAGC AGCCACTGGT AATTGATTTA GAGGAGTTAG TCTTGAAGTC    2453
   ATGCGCCGGT TAAGGCTAAA CTGAAAGGAC AAGTTTTGGT GACTGCGCTC CTCCAAGCCA    2513
   GTTACCTCGG TTCAAAGAGT TGGTAGCTCA GAGAACCTTC GAAAAACCGC CCTGCAAGGC    2573
   GGTTTTTTCG TTTTCAGAGC AAGAGATTAC GCGCAGACCA AAACGATCTC AAGAAGATCA    2633
   TCTTATTAAT CAGATAAAAT ATTTCTAGAT TTCAGTGCAA TTTATCTCTT CAAATGTAGC    2693
   ACCTGAAGTC AGCCCCATAC GATATAAGTT GTTACTAGTG CTTGGATTCT CACCAATAAA 2753
   AAACGCCCGG CGGCAACCGA GCGTTCTGAA CAAATCCAGA TGGAGTTCTG AGGTCATTAC 2813
   TGGATCTATC AACAGGAGTC CAAGCGAGCT CTCGAACCCC AGAGTCCCGC TCAGAAGAAC 2873
   TCGTCAAGAA GGCGATAGAA GGCGATGCGC TGCGAATCGG GAGCGGCGAT ACCGTAAAGC 2933
   ACGAGGAAGC GGTCAGCCCA TTCGCCGCCA AGCTCTTCAG CAATATCACG GGTAGCCAAC 2993
   GCTATGTCCT GATAGCGGTC CGCCACACCC AGCCGGCCAC AGTCGATGAA TCCAGAAAAG 3053
   CGGCCATTTT CCACCATGAT ATTCGGCAAG CAGGCATCGC CATGGGTCAC GACGAGATCC 3113
   TCGCCGTCGG GCATGCGCGC CTTGAGCCTG GCGAACAGTT CGGCTGGCGC GAGCCCCTGA 3173
   TGCTCTTCGT CCAGATCATC CTGATCGACA AGACCGGCTT CCATCCGAGT ACGTGCTCGC 3233
   TCGATGCGAT GTTTCGCTTG GTGGTCGAAT GGGCAGGTAG CCGGATCAAG CGTATGCAGC 3293
   CGCCGCATTG CATCAGCCAT GATGGATACT TTCTCGGCAG GAGCAAGGTG AGATGACAGG 3353
   AGATCCTGCC CCGGCACTTC GCCCAATAGC AGCCAGTCCC TTCCCGCTTC AGTGACAACG 3413
   TCGAGCACAG CTGCGCAAGG AACGCCCGTC GTGGCCAGCC ACGATAGCCG CGCTGCCTCG 3473
   TCCTGCAGTT CATTCAGGGC ACCGGACAGG TCGGTCTTGA CAAAAAGAAC CGGGCGCCCC 3533
   TGCGCTGACA GCCGGAACAC GGCGGCATCA GAGCAGCCGA TTGTCTGTTG TGCCCAGTCA 3593
   TAGCCGAATA GCCTCTCCAC CCAAGCGGCC GGAGAACCTG CGTGCAATCC ATCTTGTTCA 3653
   ATCATGCGAA ACGATCCTCA TCCTGTCTCT TGATCAGATC TTGATCCCCT GCGCCATCAG 3713
   ATCCTTGGCG GCAAGAAAGC CATCCAGTTT ACTTTGCAGG GCTTCCCAAC CTTACCAGAG 3773
   GGCGCCCCAG CTGGCAATTC C 3794
(2) INFORMATION ZU SEQ ID NO: 6:
   (i) SEQUENZ CHARAKTERISTIKA:
      (A) LÄNGE: 401 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 6:
   Met Asn Val Phe Asn Pro Ala Gln Phe Arg Ala Gln Phe Pro Ala Leu
   1 5 10 15
   Gln Asp Ala Gly Val Tyr Leu Asp Ser Ala Ala Thr Ala Leu Lys Pro
   20 25 30
   Glu Ala Val Val Glu Ala Thr Gln Gln Phe Tyr Ser Leu Ser Ala Gly
   35 40 45
   Asn Val His Arg Ser Gln Phe Ala Glu Ala Gln Arg Leu Thr Ala Arg
   50 55 60
   Tyr Glu Ala Ala Arg Glu Lys Val Ala Gln Leu Leu Asn Ala Pro Asp
   65 70 75 80
   Asp Lys Thr Ile Val Trp Thr Arg Gly Thr Thr Glu Ser Ile Asn Met
   85 90 95
   Val Ala Gln Cys Tyr Ala Arg Pro Arg Leu Gln Pro Gly Asp Glu Ile
   100 105 110
   Ile Val Ser Val Ala Glu His His Ala Asn Leu Val Pro Trp Leu Met
   115 120 125
   Val Ala Gln Gln Thr Gly Ala Lys Val Val Lys Leu Pro Leu Asn Ala
   130 135 140
   Gln Arg Leu Pro Asp Val Asp Leu Leu Pro Glu Leu Ile Thr Pro Arg
   145 150 155 160
   Ser Arg Ile Leu Ala Leu Gly Gln Met Ser Asn Val Thr Gly Gly Cys
   165 170 175
   Pro Asp Leu Ala Arg Ala Ile Thr Phe Ala His Ser Ala Gly Met Val
   180 185 190
   Val Met Val Asp Gly Ala Gln Gly Ala Val His Phe Pro Ala Asp Val
   195 200 205
   Gln Gln Leu Asp Ile Asp Phe Tyr Ala Phe Ser Gly His Lys Leu Tyr
   210 215 220
   Gly Pro Thr Gly Ile Gly Val Leu Tyr Gly Lys Ser Glu Leu Leu Glu
   225 230 235 240
   Ala Met Ser Pro Trp Leu Gly Gly Gly Lys Met Val His Glu Val Ser
   245 250 255
   Phe Asp Gly Phe Thr Thr Gln Ser Ala Pro Trp Lys Leu Glu Ala Gly
   260 265 270
   Thr Pro Asn Val Ala Gly Val Ile Gly Leu Ser Ala Ala Leu Glu Trp
   275 280 285
   Leu Ala Asp Tyr Asp Ile Asn Gln Ala Glu Ser Trp Ser Arg Ser Leu
   290 295 300
   Ala Thr Leu Ala Glu Asp Ala Leu Ala Lys Arg Pro Gly Phe Arg Ser
   305 310 315 320
   Phe Arg Cys Gln Asp Ser Ser Leu Leu Ala Phe Asp Phe Ala Gly Val
   325 330 335
   His His Ser Asp Met Val Thr Leu Leu Ala Glu Tyr Gly Ile Ala Leu
   340 345 350
   Arg Ala Gly Gln His Cys Ala Gln Pro Leu Leu Ala Glu Leu Gly Val
   355 360 365
   Thr Gly Thr Leu Arg Ala Ser Phe Ala Pro Tyr Asn Thr Lys Ser Asp
   370 375 380
   Val Asp Ala Leu Val Asn Ala Val Asp Arg Ala Leu Glu Leu Leu Val
   385 390 395 400
   Asp
(2) INFORMATION ZU SEQ ID NO: 7:
   (i) SEQUENZ CHARAKTERISTIKA:
      (A) LÄNGE: 3810 Basenpaare
      (B) ART: Nukleinsäure
      (C) STRANGFORM: Einzel
      (D) TOPOLOGIE: ringförmig
   (ii) ART DES MOLEKÜLS: DNS (genomisch)
   (iii) HYPOTHETISCH: NEIN
   (iii) ANTISENSE: NEIN
   (vii) UNMITTELBARE HERKUNFT:
      (B) CLON: pHS1bioS2
   (ix) MERKMALE:
      (A) NAME/SCHLÜSSEL: CDS
      (B) LAGE: 608..1822
   (xi) SEQUENZBESCHREIBUNG: SEQ ID NO: 7:
   GACGTCTGTG TGGAATTGTG AGCGGATAAC AATTTCACAC AGGGCCCTCG GACACCGAGG 60
   AGAATGTCAA GAGGCGAACA CACAACGTCT TGGAGCGCCA GAGGAGGAAC GAGCTAAAAC 120
   GGAGCTTTTT TGCCCTGCGT GACCAGATCC CGGAGTTGGA AAACAATGAA AAGGCCCCCA 180
   AGGTAGTTAT CCTTAAAAAA GCCACAGCAT ACATCCTGTC CGTCCAAGCA GAGGAGCAAA 240
   AGCTCATTTC TGAAGAGGAC TTGTTGCGGA AACGACGAGA ACAGTTGAAA CACAAACTTG 300
   AACAGCTACG GAACTCTTGT GCGTAAGGAA AAGTAAGGAA AACGATTCCT TCTAACAGAA 360
   ATGTCCTGAG CAATCACCTA TGAACTGTCG ACTCGAGATA GCATTTTTAT CCATAAGATT 420
   AGCCGATCCT AAGGTTTACA ATTGTGAGCG CTCACAATTA TGATAGATTC AATTGTGAGC      480
   GGATAACAAT TTCACACACG CTAGCGGTAC CGGGCCCCCC CTCGAGGTCG ACGGTATCGA      540
   TAAGCTTGAT ATCGAATTCC TGCAGCCCGG GGGATCCCAT GGTACGCGTA AAGAGGAGAA      600
   ATTAACT ATG AAA TTA CCG ATT TAT CTC GAC TAC TCC GCA ACC ACG CCG        649
           Met Lys Leu Pro Ile Tyr Leu Asp Tyr Ser Ala Thr Thr Pro
             1               5                  10
   GTG GAC CCG CGT GTT GCC GAG AAA ATG ATG CAG TTT ATG ACG ATG GAC        697
   Val Asp Pro Arg Val Ala Glu Lys Met Met Gln Phe Met Thr Met Asp
    15                  20                  25                  30
   GGA ACC TTT GGT AAC CCG GCC TCC CGT TCT CAC CGT TTC GGC TGG CAG        745
   Gly Thr Phe Gly Asn Pro Ala Ser Arg Ser His Arg Phe Gly Trp Gln
                    35                  40                  45
   GCT GAA GAA GCG GTA GAT ATC GCC CGT AAT CAG ATT GCC GAT CTG GTC        793
   Ala Glu Glu Ala Val Asp Ile Ala Arg Asn Gln Ile Ala Asp Leu Val
                50                  55                  60
   GGC GCT GAT CCG CGT GAA ATC GTC TTT ACC TCT GGT GCA ACC GAA TCT        841
   Gly Ala Asp Pro Arg Glu Ile Val Phe Thr Ser Gly Ala Thr Glu Ser
            65                  70                  75
   GAC AAC CTG GCG ATC AAA GGT GCA GCC AAC TTT TAT CAG AAA AAA GGC        889
   Asp Asn Leu Ala Ile Lys Gly Ala Ala Asn Phe Tyr Gln Lys Lys Gly
        80                  85                  90
   AAG CAC ATC ATC ACC AGC AAA ACC GAA CAC AAA GCG GTA CTG GAT ACC        937
   Lys His Ile Ile Thr Ser Lys Thr Glu His Lys Ala Val Leu Asp Thr
    95                 100                 105                 110
   TGC CGT CAG CTG GAG CGC GAA GGT TTT GAA GTC ACC TAC CTG GCA CCG        985
   Cys Arg Gln Leu Glu Arg Glu Gly Phe Glu Val Thr Tyr Leu Ala Pro
                   115                 120                 125
   CAG CGT AAC GGC ATT ATC GAC CTG AAA GAA CTT GAA GCA GCG ATG CGT       1033
   Gln Arg Asn Gly Ile Ile Asp Leu Lys Glu Leu Glu Ala Ala Met Arg
               130                 135                 140
   GAC GAC ACC ATC CTC GTG TCC ATC ATG CAC GTA AAT AAC GAA ATC GGC       1081
   Asp Asp Thr Ile Leu Val Ser Ile Met His Val Asn Asn Glu Ile Gly
           145                 150                 155
   GTG GTG CAG GAT ATC GCG GCT ATC GGC GAA ATG TGC CGT GCT CGT GGC       1129
   Val Val Gln Asp Ile Ala Ala Ile Gly Glu Met Cys Arg Ala Arg Gly
       160                 165                 170
   ATT ATC TAT CAC GTT GAT GCA ACC CAG AGC GTG GGT AAA CTG CCT ATC       1177
   Ile Ile Tyr His Val Asp Ala Thr Gln Ser Val Gly Lys Leu Pro Ile
   175                 180                 185                 190
   GAC CTG AGC CAG TTG AAA GTT GAC CTG ATG TCT TTC TCC GGT CAC AAA      1225
   Asp Leu Ser Gln Leu Lys Val Asp Leu Met Ser Phe Ser Gly His Lys
                       195                 200                 205
   ATC TAT GGC CCG AAA GGT ATC GGT GCG CTG TAT GTA CGT CGT AAA CCG      1273
   Ile Tyr Gly Pro Lys Gly Ile Gly Ala Leu Tyr Val Arg Arg Lys Pro
               210                 215                 220
   CGC GTA CGC ATC GAA GCG CAA ATG CAC GGC GGC GGT CAC GAG CGC GGT      1321
   Arg Val Arg Ile Glu Ala Gln Met His Gly Gly Gly His Glu Arg Gly
           225                 230                 235
   ATG CGT TCC GGC ACT CTG CCT GTT CAC CAG ATC GTC GGA ATG GGC GAG      1369
   Met Arg Ser Gly Thr Leu Pro Val His Gln Ile Val Gly Met Gly Glu
       240                 245                 250
   GCC TAT CGC ATC GCA AAA GAA GAG ATG GCG ACC GAG ATG GAA CGT CTG      1417
   Ala Tyr Arg Ile Ala Lys Glu Glu Met Ala Thr Glu Met Glu Arg Leu
   255                 260                 265                 270
   CGC GGC CTG CGT AAC CGT CTG TGG AAC GGC ATC AAA GAT ATC GAA GAA      1465
   Arg Gly Leu Arg Asn Arg Leu Trp Asn Gly Ile Lys Asp Ile Glu Glu
                       275                 280                 285
   GTT TAC CTG AAC GGT GAC CTG GAA CAC GGT GCG CCG AAC ATT CTC AAC      1513
   Val Tyr Leu Asn Gly Asp Leu Glu His Gly Ala Pro Asn Ile Leu Asn
               290                 295                 300
   GTC AGC TTC AAC TAC GTT GAA GGT GAG TCG CTG ATT ATG GCG CTG AAA      1561
   Val Ser Phe Asn Tyr Val Glu Gly Glu Ser Leu Ile Met Ala Leu Lys
           305                 310                 315
   GAC CTC GCA GTT TCT TCA GGT TCC GCC TGT ACG TCA GCA AGC CTC GAA      1609
   Asp Leu Ala Val Ser Ser Gly Ser Ala Cys Thr Ser Ala Ser Leu Glu
       320                 325                 330
   CCG TCC TAC GTG CTG CGC GCG CTG GGG CTG AAC GAC GAG CTG GCA CAT      1657
   Pro Ser Tyr Val Leu Arg Ala Leu Gly Leu Asn Asp Glu Leu Ala His
   335                 340                 345                 350
   AGC TCT ATC CGT TTC TCT TTA GGT CGT TTT ACT ACT GAA GAA GAG ATC      1705
   Ser Ser Ile Arg Phe Ser Leu Gly Arg Phe Thr Thr Glu Glu Glu Ile
                       355                 360                 365
   GAC TAC ACC ATC GAG TTA GTT CGT AAA TCC ATC GGT CGT CTG CGT GAC      1753
   Asp Tyr Thr Ile Glu Leu Val Arg Lys Ser Ile Gly Arg Leu Arg Asp
               370                 375                 380
   CTT TCT CCG CTG TGG GAA ATG TAC AAG CAG GGC GTG GAT CTG AAC AGC      1801
   Leu Ser Pro Leu Trp Glu Met Tyr Lys Gln Gly Val Asp Leu Asn Ser
           385                 390                 395
   ATC GAA TGG GCT CAT CAT TAAACGCGTG CTAGAGGCAT CAAATAAAAC              1849
   Ile Glu Trp Ala His His
       400                 405
   GAAAGGCTCA GTCGAAAGAC TGGGCCTTTC GTTTTATCTG TTGTTTGTCG GTGAACGCTC    1909
   TCCTGAGTAG GACAAATCCG CCGCCCTAGA CCTAGGGGAT ATATTCCGCT TCCTCGCTCA    1969
   CTGACTCGCT ACGCTCGGTC GTTCGACTGC GGCGAGCGGA AATGGCTTAC GAACGGGGCG    2029
   GAGATTTCCT GGAAGATGCC AGGAAGATAC TTAACAGGGA AGTGAGAGGG CCGCGGCAAA    2089
   GCCGTTTTTC CATAGGCTCC GCCCCCCTGA CAAGCATCAC GAAATCTGAC GCTCAAATCA    2149
   GTGGTGGCGA AACCCGACAG GACTATAAAG ATACCAGGCG TTTCCCCCTG GCGGCTCCCT    2209
   CGTGCGCTCT CCTGTTCCTG CCTTTCGGTT TACCGGTGTC ATTCCGCTGT TATGGCCGCG    2269
   TTTGTCTCAT TCCACGCCTG ACACTCAGTT CCGGGTAGGC AGTTCGCTCC AAGCTGGACT    2329
   GTATGCACGA ACCCCCCGTT CAGTCCGACC GCTGCGCCTT ATCCGGTAAC TATCGTCTTG    2389
   AGTCCAACCC GGAAAGACAT GCAAAAGCAC CACTGGCAGC AGCCACTGGT AATTGATTTA    2449
   GAGGAGTTAG TCTTGAAGTC ATGCGCCGGT TAAGGCTAAA CTGAAAGGAC AAGTTTTGGT    2509
   GACTGCGCTC CTCCAAGCCA GTTACCTCGG TTCAAAGAGT TGGTAGCTCA GAGAACCTTC    2569
   GAAAAACCGC CCTGCAAGGC GGTTTTTTCG TTTTCAGAGC AAGAGATTAC GCGCAGACCA    2629
   AAACGATCTC AAGAAGATCA TCTTATTAAT CAGATAAAAT ATTTCTAGAT TTCAGTGCAA    2689
   TTTATCTCTT CAAATGTAGC ACCTGAAGTC AGCCCCATAC GATATAAGTT GTTACTAGTG    2749
   CTTGGATTCT CACCAATAAA AAACGCCCGG CGGCAACCGA GCGTTCTGAA CAAATCCAGA    2809
   TGGAGTTCTG AGGTCATTAC TGGATCTATC AACAGGAGTC CAAGCGAGCT CTCGAACCCC    2869
   AGAGTCCCGC TCAGAAGAAC TCGTCAAGAA GGCGATAGAA GGCGATGCGC TGCGAATCGG    2929
   GAGCGGCGAT ACCGTAAAGC ACGAGGAAGC GGTCAGCCCA TTCGCCGCCA AGCTCTTCAG    2989
   CAATATCACG GGTAGCCAAC GCTATGTCCT GATAGCGGTC CGCCACACCC AGCCGGCCAC    3049
   AGTCGATGAA TCCAGAAAAG CGGCCATTTT CCACCATGAT ATTCGGCAAG CAGGCATCGC    3109
   CATGGGTCAC GACGAGATCC TCGCCGTCGG GCATGCGCGC CTTGAGCCTG GCGAACAGTT    3169
   CGGCTGGCGC GAGCCCCTGA TGCTCTTCGT CCAGATCATC CTGATCGACA AGACCGGCTT    3229
   CCATCCGAGT ACGTGCTCGC TCGATGCGAT GTTTCGCTTG GTGGTCGAAT GGGCAGGTAG    3289
   CCGGATCAAG CGTATGCAGC CGCCGCATTG CATCAGCCAT GATGGATACT TTCTCGGCAG    3349
   GAGCAAGGTG AGATGACAGG AGATCCTGCC CCGGCACTTC GCCCAATAGC AGCCAGTCCC    3409
   TTCCCGCTTC AGTGACAACG TCGAGCACAG CTGCGCAAGG AACGCCCGTC GTGGCCAGCC    3469
   ACGATAGCCG CGCTGCCTCG TCCTGCAGTT CATTCAGGGC ACCGGACAGG TCGGTCTTGA 3529
   CAAAAAGAAC CGGGCGCCCC TGCGCTGACA GCCGGAACAC GGCGGCATCA GAGCAGCCGA 3589
   TTGTCTGTTG TGCCCAGTCA TAGCCGAATA GCCTCTCCAC CCAAGCGGCC GGAGAACCTG 3649
   CGTGCAATCC ATCTTGTTCA ATCATGCGAA ACGATCCTCA TCCTGTCTCT TGATCAGATC 3709
   TTGATCCCCT GCGCCATCAG ATCCTTGGCG GCAAGAAAGC CATCCAGTTT ACTTTGCAGG 3769
   GCTTCCCAAC CTTACCAGAG GGCGCCCCAG CTGGCAATTC C 3810
(2) INFORMATION ZU SEQ ID NO: 8:
   (i) SEQUENZ CHARAKTERISTIKA:
      (A) LÄNGE: 404 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 8:
   Met Lys Leu Pro Ile Tyr Leu Asp Tyr Ser Ala Thr Thr Pro Val Asp
    1               5                  10                  15
   Pro Arg Val Ala Glu Lys Met Met Gln Phe Met Thr Met Asp Gly Thr
               20                  25                  30
   Phe Gly Asn Pro Ala Ser Arg Ser His Arg Phe Gly Trp Gln Ala Glu
           35                  40                  45
   Glu Ala Val Asp Ile Ala Arg Asn Gln Ile Ala Asp Leu Val Gly Ala
       50                  55                  60
   Asp Pro Arg Glu Ile Val Phe Thr Ser Gly Ala Thr Glu Ser Asp Asn
   65                  70                  75                  80
   Leu Ala Ile Lys Gly Ala Ala Asn Phe Tyr Gln Lys Lys Gly Lys His
                   85                  90                  95
   Ile Ile Thr Ser Lys Thr Glu His Lys Ala Val Leu Asp Thr Cys Arg
               100                 105                 110
   Gln Leu Glu Arg Glu Gly Phe Glu Val Thr Tyr Leu Ala Pro Gln Arg
           115                 120                 125
   Asn Gly Ile Ile Asp Leu Lys Glu Leu Glu Ala Ala Met Arg Asp Asp
       130                 135                 140
   Thr Ile Leu Val Ser Ile Met His Val Asn Asn Glu Ile Gly Val Val
   145                 150                 155                 160
   Gln Asp Ile Ala Ala Ile Gly Glu Met Cys Arg Ala Arg Gly Ile Ile
                   165                 170                 175
   Tyr His Val Asp Ala Thr Gln Ser Val Gly Lys Leu Pro Ile Asp Leu
               180                 185                 190
   Ser Gln Leu Lys Val Asp Leu Met Ser Phe Ser Gly His Lys Ile Tyr
           195                 200                 205
   Gly Pro Lys Gly Ile Gly Ala Leu Tyr Val Arg Arg Lys Pro Arg Val
       210                 215                 220
   Arg Ile Glu Ala Gln Met His Gly Gly Gly His Glu Arg Gly Met Arg
   225                 230                 235                 240
   Ser Gly Thr Leu Pro Val His Gln Ile Val Gly Met Gly Glu Ala Tyr
                   245                 250                 255
   Arg Ile Ala Lys Glu Glu Met Ala Thr Glu Met Glu Arg Leu Arg Gly
               260                 265                 270
   Leu Arg Asn Arg Leu Trp Asn Gly Ile Lys Asp Ile Glu Glu Val Tyr
           275                 280                 285
   Leu Asn Gly Asp Leu Glu His Gly Ala Pro Asn Ile Leu Asn Val Ser
       290                 295                 300
   Phe Asn Tyr Val Glu Gly Glu Ser Leu Ile Met Ala Leu Lys Asp Leu
   305                 310                 315                 320
   Ala Val Ser Ser Gly Ser Ala Cys Thr Ser Ala Ser Leu Glu Pro Ser
                   325                 330                 335
   Tyr Val Leu Arg Ala Leu Gly Leu Asn Asp Glu Leu Ala His Ser Ser
               340                 345                 350
   Ile Arg Phe Ser Leu Gly Arg Phe Thr Thr Glu Glu Glu Ile Asp Tyr
           355                 360                 365
   Thr Ile Glu Leu Val Arg Lys Ser Ile Gly Arg Leu Arg Asp Leu Ser
       370                 375                 380
   Pro Leu Trp Glu Met Tyr Lys Gln Gly Val Asp Leu Asn Ser Ile Glu
   385                 390                 395                 400
   Trp Ala His His
(2) INFORMATION ZU SEQ ID NO: 9:
   (i) SEQUENZ CHARAKTERISTIKA:
      (A) LÄNGE: 3465 Basenpaare
      (B) ART: Nukleinsäure
      (C) STRANGFORM: Einzel
      (D) TOPOLOGIE: ringförmig
   (ii) ART DES MOLEKÜLS: DNS (genomisch)
   (iii) HYPOTHETISCH: NEIN
   (iii) ANTISENSE: NEIN
   (vii) UNMITTELBARE HERKUNFT:
      (B) CLON: pHS2bioS1
   (ix) MERKMALE:
      (A) NAME/SCHLÜSSEL: CDS
      (B) LAGE: 272..1477
   (xi) SEQUENZBESCHREIBUNG: SEQ ID NO: 9:
   GACGTCTAAG AAACCATTAT TATCATGACA TTAACCTATA AAAATAGGCG TATCACGAGG      60
   CCCTTTCGTC TTCACCTCGA GTCCCTATCA GTGATAGAGA TTGACATCCC TATCAGTGAT     120
   AGAGATACTG AGCACATCAG CAGGACGCAC TGACCGAATT CATTAAAGAG GAGAAAGGTA     180
   CCGGGCCCCC CCTCGAGGTC GACGGTATCG ATAAGCTTGA TATCGAATTC CTGCAGCCCG     240
   GGGGATCCCA TGGTACGCGT CGAGGAGTAC C ATG AAC GTT TTT AAT CCC GCG        292
                                      Met Asn Val Phe Asn Pro Ala
                                        1               5
   CAG TTT CGC GCC CAG TTT CCC GCA CTA CAG GAT GCG GGC GTC TAT CTC      340
   Gln Phe Arg Ala Gln Phe Pro Ala Leu Gln Asp Ala Gly Val Tyr Leu
           10                  15                  20
   GAC AGC GCC GCG ACC GCG CTT AAA CCT GAA GCC GTG GTT GAA GCC ACC      388
   Asp Ser Ala Ala Thr Ala Leu Lys Pro Glu Ala Val Val Glu Ala Thr
       25                  30                  35
   CAA CAG TTT TAC AGT CTG AGC GCC GGA AAC GTC CAT CGC AGC CAG TTT      436
   Gln Gln Phe Tyr Ser Leu Ser Ala Gly Asn Val His Arg Ser Gln Phe
    40                  45                  50                  55
   GCC GAA GCC CAA CGC CTG ACC GCG CGT TAT GAA GCT GCA CGA GAG AAA      484
   Ala Glu Ala Gln Arg Leu Thr Ala Arg Tyr Glu Ala Ala Arg Glu Lys
                    60                  65                  70
   GTG GCG CAA TTA CTG AAT GCA CCG GAT GAT AAA ACT ATC GTC TGG ACG      532
   Val Ala Gln Leu Leu Asn Ala Pro Asp Asp Lys Thr Ile Val Trp Thr
                75                  80                  85
   CGC GGC ACC ACT GAA TCC ATC AAC ATG GTG GCA CAA TGC TAT GCG CGT      580
   Arg Gly Thr Thr Glu Ser Ile Asn Met Val Ala Gln Cys Tyr Ala Arg
           90                  95                 100
   CCG CGT CTG CAA CCG GGC GAT GAG ATT ATT GTC AGC GTG GCA GAA CAC      628
   Pro Arg Leu Gln Pro Gly Asp Glu Ile Ile Val Ser Val Ala Glu His
       105                 110                 115
   CAC GCC AAC CTC GTC CCC TGG CTG ATG GTC GCC CAA CAA ACT GGA GCC 676
   His Ala Asn Leu Val Pro Trp Leu Met Val Ala Gln Gln Thr Gly Ala
   120 125 130 135
   AAA GTG GTG AAA TTG CCG CTT AAT GCG CAG CGA CTG CCG GAT GTC GAT 724
   Lys Val Val Lys Leu Pro Leu Asn Ala Gln Arg Leu Pro Asp Val Asp
   140 145 150
   TTG TTG CCA GAA CTG ATT ACT CCC CGT AGT CGG ATT CTG GCG TTG GGT 772
   Leu Leu Pro Glu Leu Ile Thr Pro Arg Ser Arg Ile Leu Ala Leu Gly
   155 160 165
   CAG ATG TCG AAC GTT ACT GGC GGT TGC CCG GAT CTG GCG CGA GCG ATT 820
   Gln Met Ser Asn Val Thr Gly Gly Cys Pro Asp Leu Ala Arg Ala Ile
   170 175 180
   ACC TTT GCT CAT TCA GCC GGG ATG GTG GTG ATG GTT GAT GGT GCT CAG 868
   Thr Phe Ala His Ser Ala Gly Met Val Val Met Val Asp Gly Ala Gln
   185 190 195
   GGG GCA GTG CAT TTC CCC GCG GAT GTT CAG CAA CTG GAT ATT GAT TTC 916
   Gly Ala Val His Phe Pro Ala Asp Val Gln Gln Leu Asp Ile Asp Phe
   200 205 210 215
   TAT GCT TTT TCA GGT CAC AAA CTG TAT GGC CCG ACA GGT ATC GGC GTG 964
   Tyr Ala Phe Ser Gly His Lys Leu Tyr Gly Pro Thr Gly Ile Gly Val
   220 225 230
   CTG TAT GGT AAA TCA GAA CTG CTG GAG GCG ATG TCG CCC TGG CTG GGC 1012
   Leu Tyr Gly Lys Ser Glu Leu Leu Glu Ala Met Ser Pro Trp Leu Gly
   235 240 245
   GGC GGC AAA ATG GTT CAC GAA GTG AGT TTT GAC GGC TTC ACG ACT CAA 1060
   Gly Gly Lys Met Val His Glu Val Ser Phe Asp Gly Phe Thr Thr Gln
   250 255 260
   TCT GCG CCG TGG AAA CTG GAA GCT GGA ACG CCA AAT GTC GCT GGT GTC 1108
   Ser Ala Pro Trp Lys Leu Glu Ala Gly Thr Pro Asn Val Ala Gly Val
   265 270 275
   ATA GGA TTA AGC GCG GCG CTG GAA TGG CTG GCA GAT TAC GAT ATC AAC 1156
   Ile Gly Leu Ser Ala Ala Leu Glu Trp Leu Ala Asp Tyr Asp Ile Asn
   280 285 290 295
   CAG GCC GAA AGC TGG AGC CGT AGC TTA GCA ACG CTG GCG GAA GAT GCG 1204
   Gln Ala Glu Ser Trp Ser Arg Ser Leu Ala Thr Leu Ala Glu Asp Ala
   300 305 310
   CTG GCG AAA CGT CCC GGC TTT CGT TCA TTC CGC TGC CAG GAT TCC AGC 1252
   Leu Ala Lys Arg Pro Gly Phe Arg Ser Phe Arg Cys Gln Asp Ser Ser
   315 320 325
   CTG CTG GCC TTT GAT TTT GCT GGC GTT CAT CAT AGC GAT ATG GTG ACG      1300
   Leu Leu Ala Phe Asp Phe Ala Gly Val His His Ser Asp Met Val Thr
           330                 335                     340
   CTG CTG GCG GAG TAC GGT ATT GCC CTG CGG GCC GGG CAG CAT TGC GCT      1348
   Leu Leu Ala Glu Tyr Gly Ile Ala Leu Arg Ala Gly Gln His Cys Ala
       345                 350                     355
   CAG CCG CTA CTG GCA GAA TTA GGC GTA ACC GGC ACA CTG CGC GCC TCT      1396
   Gln Pro Leu Leu Ala Glu Leu Gly Val Thr Gly Thr Leu Arg Ala Ser
   360                 365                     370                 375
   TTT GCG CCA TAT AAT ACA AAG AGT GAT GTG GAT GCG CTG GTG AAT GCC      1444
   Phe Ala Pro Tyr Asn Thr Lys Ser Asp Val Asp Ala Leu Val Asn Ala
                   380                     385                 390
   GTT GAC CGC GCG CTG GAA TTA TTG GTG GAT TAAACGCGTG CTAGAGGCAT        1494
   Val Asp Arg Ala Leu Glu Leu Leu Val Asp
               395                     400
   CAAATAAAAC GAAAGGCTCA GTCGAAAGAC TGGGCCTTTC GTTTTATCTG TTGTTTGTCG    1554
   GTGAACGCTC TCCTGAGTAG GACAAATCCG CCGCCCTAGA CCTAGGGGAT ATATTCCGCT    1614
   TCCTCGCTCA CTGACTCGCT ACGCTCGGTC GTTCGACTGC GGCGAGCGGA AATGGCTTAC    1674
   GAACGGGGCG GAGATTTCCT GGAAGATGCC AGGAAGATAC TTAACAGGGA AGTGAGAGGG    1734
   CCGCGGCAAA GCCGTTTTTC CATAGGCTCC GCCCCCCTGA CAAGCATCAC GAAATCTGAC    1794
   GCTCAAATCA GTGGTGGCGA AACCCGACAG GACTATAAAG ATACCAGGCG TTTCCCCCTG    1854
   GCGGCTCCCT CGTGCGCTCT CCTGTTCCTG CCTTTCGGTT TACCGGTGTC ATTCCGCTGT    1914
   TATGGCCGCG TTTGTCTCAT TCCACGCCTG ACACTCAGTT CCGGGTAGGC AGTTCGCTCC    1974
   AAGCTGGACT GTATGCACGA ACCCCCCGTT CAGTCCGACC GCTGCGCCTT ATCCGGTAAC    2034
   TATCGTCTTG AGTCCAACCC GGAAAGACAT GCAAAAGCAC CACTGGCAGC AGCCACTGGT    2094
   AATTGATTTA GAGGAGTTAG TCTTGAAGTC ATGCGCCGGT TAAGGCTAAA CTGAAAGGAC    2154
   AAGTTTTGGT GACTGCGCTC CTCCAAGCCA GTTACCTCGG TTCAAAGAGT TGGTAGCTCA    2214
   GAGAACCTTC GAAAAACCGC CCTGCAAGGC GGTTTTTTCG TTTTCAGAGC AAGAGATTAC    2274
   GCGCAGACCA AAACGATCTC AAGAAGATCA TCTTATTAAT CAGATAAAAT ATTTCTAGAT    2334
   TTCAGTGCAA TTTATCTCTT CAAATGTAGC ACCTGAAGTC AGCCCCATAC GATATAAGTT    2394
   GTTACTAGTG CTTGGATTCT CACCAATAAA AAACGCCCGG CGGCAACCGA GCGTTCTGAA    2454
   CAAATCCAGA TGGAGTTCTG AGGTCATTAC TGGATCTATC AACAGGAGTC CAAGCGAGCT    2514
   CTCGAACCCC AGAGTCCCGC TCAGAAGAAC TCGTCAAGAA GGCGATAGAA GGCGATGCGC    2574
   TGCGAATCGG GAGCGGCGAT ACCGTAAAGC ACGAGGAAGC GGTCAGCCCA TTCGCCGCCA 2634
   AGCTCTTCAG CAATATCACG GGTAGCCAAC GCTATGTCCT GATAGCGGTC CGCCACACCC 2694
   AGCCGGCCAC AGTCGATGAA TCCAGAAAAG CGGCCATTTT CCACCATGAT ATTCGGCAAG 2754
   CAGGCATCGC CATGGGTCAC GACGAGATCC TCGCCGTCGG GCATGCGCGC CTTGAGCCTG 2814
   GCGAACAGTT CGGCTGGCGC GAGCCCCTGA TGCTCTTCGT CCAGATCATC CTGATCGACA 2874
   AGACCGGCTT CCATCCGAGT ACGTGCTCGC TCGATGCGAT GTTTCGCTTG GTGGTCGAAT 2934
   GGGCAGGTAG CCGGATCAAG CGTATGCAGC CGCCGCATTG CATCAGCCAT GATGGATACT 2994
   TTCTCGGCAG GAGCAAGGTG AGATGACAGG AGATCCTGCC CCGGCACTTC GCCCAATAGC 3054
   AGCCAGTCCC TTCCCGCTTC AGTGACAACG TCGAGCACAG CTGCGCAAGG AACGCCCGTC 3114
   GTGGCCAGCC ACGATAGCCG CGCTGCCTCG TCCTGCAGTT CATTCAGGGC ACCGGACAGG 3174
   TCGGTCTTGA CAAAAAGAAC CGGGCGCCCC TGCGCTGACA GCCGGAACAC GGCGGCATCA 3234
   GAGCAGCCGA TTGTCTGTTG TGCCCAGTCA TAGCCGAATA GCCTCTCCAC CCAAGCGGCC 3294
   GGAGAACCTG CGTGCAATCC ATCTTGTTCA ATCATGCGAA ACGATCCTCA TCCTGTCTCT 3354
   TGATCAGATC TTGATCCCCT GCGCCATCAG ATCCTTGGCG GCAAGAAAGC CATCCAGTTT 3414
   ACTTTGCAGG GCTTCCCAAC CTTACCAGAG GGCGCCCCAG CTGGCAATTC C 3465
(2) INFORMATION ZU SEQ ID NO: 10:
   (i) SEQUENZ CHARAKTERISTIKA:
      (A) LÄNGE: 401 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 10:
   Met Asn Val Phe Asn Pro Ala Gln Phe Arg Ala Gln Phe Pro Ala Leu
   1 5 10 15
   Gln Asp Ala Gly Val Tyr Leu Asp Ser Ala Ala Thr Ala Leu Lys Pro
   20 25 30
   Glu Ala Val Val Glu Ala Thr Gln Gln Phe Tyr Ser Leu Ser Ala Gly
   35 40 45
   Asn Val His Arg Ser Gln Phe Ala Glu Ala Gln Arg Leu Thr Ala Arg
   50 55 60
   Tyr Glu Ala Ala Arg Glu Lys Val Ala Gln Leu Leu Asn Ala Pro Asp
   65 70 75 80
   Asp Lys Thr Ile Val Trp Thr Arg Gly Thr Thr Glu Ser Ile Asn Met
   85 90 95
   Val Ala Gln Cys Tyr Ala Arg Pro Arg Leu Gln Pro Gly Asp Glu Ile
   100 105 110
   Ile Val Ser Val Ala Glu His His Ala Asn Leu Val Pro Trp Leu Met
   115 120 125
   Val Ala Gln Gln Thr Gly Ala Lys Val Val Lys Leu Pro Leu Asn Ala
   130 135 140
   Gln Arg Leu Pro Asp Val Asp Leu Leu Pro Glu Leu Ile Thr Pro Arg
   145 150 155 160
   Ser Arg Ile Leu Ala Leu Gly Gln Met Ser Asn Val Thr Gly Gly Cys
   165 170 175
   Pro Asp Leu Ala Arg Ala Ile Thr Phe Ala His Ser Ala Gly Met Val
   180 185 190
   Val Met Val Asp Gly Ala Gln Gly Ala Val His Phe Pro Ala Asp Val
   195 200 205
   Gln Gln Leu Asp Ile Asp Phe Tyr Ala Phe Ser Gly His Lys Leu Tyr
   210 215 220
   Gly Pro Thr Gly Ile Gly Val Leu Tyr Gly Lys Ser Glu Leu Leu Glu
   225 230 235 240
   Ala Met Ser Pro Trp Leu Gly Gly Gly Lys Met Val His Glu Val Ser
   245 250 255
   Phe Asp Gly Phe Thr Thr Gln Ser Ala Pro Trp Lys Leu Glu Ala Gly
   260 265 270
   Thr Pro Asn Val Ala Gly Val Ile Gly Leu Ser Ala Ala Leu Glu Trp
   275 280 285
   Leu Ala Asp Tyr Asp Ile Asn Gln Ala Glu Ser Trp Ser Arg Ser Leu
   290 295 300
   Ala Thr Leu Ala Glu Asp Ala Leu Ala Lys Arg Pro Gly Phe Arg Ser
   305 310 315 320
   Phe Arg Cys Gln Asp Ser Ser Leu Leu Ala Phe Asp Phe Ala Gly Val
   325 330 335
   His His Ser Asp Met Val Thr Leu Leu Ala Glu Tyr Gly Ile Ala Leu
   340 345 350
   Arg Ala Gly Gln His Cys Ala Gln Pro Leu Leu Ala Glu Leu Gly Val
   355 360 365
   Thr Gly Thr Leu Arg Ala Ser Phe Ala Pro Tyr Asn Thr Lys Ser Asp
       370                 375                 380
   Val Asp Ala Leu Val Asn Ala Val Asp Arg Ala Leu Glu Leu Leu Val
   385                 390                 395                 400
   Asp
(2) INFORMATION ZU SEQ ID NO: 11:
   (i) SEQUENZ CHARAKTERISTIKA:
      (A) LÄNGE: 3481 Basenpaare
      (B) ART: Nukleinsäure
      (C) STRANGFORM: Einzel
      (D) TOPOLOGIE: ringförmig
   (ii) ART DES MOLEKÜLS: DNS (genomisch)
   (iii) HYPOTHETISCH: NEIN
   (iii) ANTISENSE: NEIN
   (vii) UNMITTELBARE HERKUNFT:
      (B) CLON: pHS2bioS2
   (ix) MERKMALE:
      (A) NAME/SCHLÜSSEL: CDS
      (B) LAGE: 279..1493
   (xi) SEQUENZBESCHREIBUNG: SEQ ID NO: 11:
   GACGTCTAAG AAACCATTAT TATCATGACA TTAACCTATA AAAATAGGCG TATCACGAGG      60
   CCCTTTCGTC TTCACCTCGA GTCCCTATCA GTGATAGAGA TTGACATCCC TATCAGTGAT     120
   AGAGATACTG AGCACATCAG CAGGACGCAC TGACCGAATT CATTAAAGAG GAGAAAGGTA     180
   CCGGGCCCCC CCTCGAGGTC GACGGTATCG ATAAGCTTGA TATCGAATTC CTGCAGCCCG     240
   GGGGATCCCA TGGTACGCGT AAAGAGGAGA AATTAACT ATG AAA TTA CCG ATT        293
                                            Met Lys Leu Pro Ile
                                             1               5
   TAT CTC GAC TAC TCC GCA ACC ACG CCG GTG GAC CCG CGT GTT GCC GAG      341
   Tyr Leu Asp Tyr Ser Ala Thr Thr Pro Val Asp Pro Arg Val Ala Glu
                    10                  15                  20
   AAA ATG ATG CAG TTT ATG ACG ATG GAC GGA ACC TTT GGT AAC CCG GCC      389
   Lys Met Met Gln Phe Met Thr Met Asp Gly Thr Phe Gly Asn Pro Ala
                25                  30                  35
   TCC CGT TCT CAC CGT TTC GGC TGG CAG GCT GAA GAA GCG GTA GAT ATC      437
   Ser Arg Ser His Arg Phe Gly Trp Gln Ala Glu Glu Ala Val Asp Ile
           40                  45                  50
   GCC CGT AAT CAG ATT GCC GAT CTG GTC GGC GCT GAT CCG CGT GAA ATC      485
   Ala Arg Asn Gln Ile Ala Asp Leu Val Gly Ala Asp Pro Arg Glu Ile
       55                  60                  65
   GTC TTT ACC TCT GGT GCA ACC GAA TCT GAC AAC CTG GCG ATC AAA GGT      533
   Val Phe Thr Ser Gly Ala Thr Glu Ser Asp Asn Leu Ala Ile Lys Gly
   70                  75                  80                  85
   GCA GCC AAC TTT TAT CAG AAA AAA GGC AAG CAC ATC ATC ACC AGC AAA      581
   Ala Ala Asn Phe Tyr Gln Lys Lys Gly Lys His Ile Ile Thr Ser Lys
                   90                  95                  100
   ACC GAA CAC AAA GCG GTA CTG GAT ACC TGC CGT CAG CTG GAG CGC GAA      629
   Thr Glu His Lys Ala Val Leu Asp Thr Cys Arg Gln Leu Glu Arg Glu
               105                 110                 115
   GGT TTT GAA GTC ACC TAC CTG GCA CCG CAG CGT AAC GGC ATT ATC GAC      677
   Gly Phe Glu Val Thr Tyr Leu Ala Pro Gln Arg Asn Gly Ile Ile Asp
           120                 125                 130
   CTG AAA GAA CTT GAA GCA GCG ATG CGT GAC GAC ACC ATC CTC GTG TCC      725
   Leu Lys Glu Leu Glu Ala Ala Met Arg Asp Asp Thr Ile Leu Val Ser
       135                 140                 145
   ATC ATG CAC GTA AAT AAC GAA ATC GGC GTG GTG CAG GAT ATC GCG GCT      773
   Ile Met His Val Asn Asn Glu Ile Gly Val Val Gln Asp Ile Ala Ala
   150                 155                 160                 165
   ATC GGC GAA ATG TGC CGT GCT CGT GGC ATT ATC TAT CAC GTT GAT GCA      821
   Ile Gly Glu Met Cys Arg Ala Arg Gly Ile Ile Tyr His Val Asp Ala
                   170                 175                 180
   ACC CAG AGC GTG GGT AAA CTG CCT ATC GAC CTG AGC CAG TTG AAA GTT      869
   Thr Gln Ser Val Gly Lys Leu Pro Ile Asp Leu Ser Gln Leu Lys Val
               185                 190                 195
   GAC CTG ATG TCT TTC TCC GGT CAC AAA ATC TAT GGC CCG AAA GGT ATC      917
   Asp Leu Met Ser Phe Ser Gly His Lys Ile Tyr Gly Pro Lys Gly Ile
           200                 205                 210
   GGT GCG CTG TAT GTA CGT CGT AAA CCG CGC GTA CGC ATC GAA GCG CAA      965
   Gly Ala Leu Tyr Val Arg Arg Lys Pro Arg Val Arg Ile Glu Ala Gln
       215                 220                 225
   ATG CAC GGC GGC GGT CAC GAG CGC GGT ATG CGT TCC GGC ACT CTG CCT     1013
   Met His Gly Gly Gly His Glu Arg Gly Met Arg Ser Gly Thr Leu Pro
   230                 235                 240                 245
   GTT CAC CAG ATC GTC GGA ATG GGC GAG GCC TAT CGC ATC GCA AAA GAA      1061
   Val His Gln Ile Val Gly Met Gly Glu Ala Tyr Arg Ile Ala Lys Glu
                   250                     255                     260
   GAG ATG GCG ACC GAG ATG GAA CGT CTG CGC GGC CTG CGT AAC CGT CTG      1109
   Glu Met Ala Thr Glu Met Glu Arg Leu Arg Gly Leu Arg Asn Arg Leu
               265                     270                     275
   TGG AAC GGC ATC AAA GAT ATC GAA GAA GTT TAC CTG AAC GGT GAC CTG      1157
   Trp Asn Gly Ile Lys Asp Ile Glu Glu Val Tyr Leu Asn Gly Asp Leu
           280                     285                     290
   GAA CAC GGT GCG CCG AAC ATT CTC AAC GTC AGC TTC AAC TAC GTT GAA      1205
   Glu His Gly Ala Pro Asn Ile Leu Asn Val Ser Phe Asn Tyr Val Glu
       295                     300                     305
   GGT GAG TCG CTG ATT ATG GCG CTG AAA GAC CTC GCA GTT TCT TCA GGT      1253
   Gly Glu Ser Leu Ile Met Ala Leu Lys Asp Leu Ala Val Ser Ser Gly
   310                     315                     320                     325
   TCC GCC TGT ACG TCA GCA AGC CTC GAA CCG TCC TAC GTG CTG CGC GCG      1301
   Ser Ala Cys Thr Ser Ala Ser Leu Glu Pro Ser Tyr Val Leu Arg Ala
                   330                     335                     340
   CTG GGG CTG AAC GAC GAG CTG GCA CAT AGC TCT ATC CGT TTC TCT TTA      1349
   Leu Gly Leu Asn Asp Glu Leu Ala His Ser Ser Ile Arg Phe Ser Leu
               345                     350                     355
   GGT CGT TTT ACT ACT GAA GAA GAG ATC GAC TAC ACC ATC GAG TTA GTT      1397
   Gly Arg Phe Thr Thr Glu Glu Glu Ile Asp Tyr Thr Ile Glu Leu Val
           360                     365                     370
   CGT AAA TCC ATC GGT CGT CTG CGT GAC CTT TCT CCG CTG TGG GAA ATG      1445
   Arg Lys Ser Ile Gly Arg Leu Arg Asp Leu Ser Pro Leu Trp Glu Met
       375                     380                     385
   TAC AAG CAG GGC GTG GAT CTG AAC AGC ATC GAA TGG GCT CAT CAT TAAACGCGTG    1500
   Tyr Lys Gln Gly Val Asp Leu Asn Ser Ile Glu Trp Ala His His
   390                     395                     400                     405
   CTAGAGGCAT CAAATAAAAC GAAAGGCTCA GTCGAAAGAC TGGGCCTTTC GTTTTATCTG      1560
   TTGTTTGTCG GTGAACGCTC TCCTGAGTAG GACAAATCCG CCGCCCTAGA CCTAGGGGAT      1620
   ATATTCCGCT TCCTCGCTCA CTGACTCGCT ACGCTCGGTC GTTCGACTGC GGCGAGCGGA      1680
   AATGGCTTAC GAACGGGGCG GAGATTTCCT GGAAGATGCC AGGAAGATAC TTAACAGGGA      1740
   AGTGAGAGGG CCGCGGCAAA GCCGTTTTTC CATAGGCTCC GCCCCCCTGA CAAGCATCAC      1800
   GAAATCTGAC GCTCAAATCA GTGGTGGCGA AACCCGACAG GACTATAAAG ATACCAGGCG      1860
   TTTCCCCCTG GCGGCTCCCT CGTGCGCTCT CCTGTTCCTG CCTTTCGGTT TACCGGTGTC      1920
   ATTCCGCTGT TATGGCCGCG TTTGTCTCAT TCCACGCCTG ACACTCAGTT CCGGGTAGGC 1980
   AGTTCGCTCC AAGCTGGACT GTATGCACGA ACCCCCCGTT CAGTCCGACC GCTGCGCCTT 2040
   ATCCGGTAAC TATCGTCTTG AGTCCAACCC GGAAAGACAT GCAAAAGCAC CACTGGCAGC 2100
   AGCCACTGGT AATTGATTTA GAGGAGTTAG TCTTGAAGTC ATGCGCCGGT TAAGGCTAAA 2160
   CTGAAAGGAC AAGTTTTGGT GACTGCGCTC CTCCAAGCCA GTTACCTCGG TTCAAAGAGT 2220
   TGGTAGCTCA GAGAACCTTC GAAAAACCGC CCTGCAAGGC GGTTTTTTCG TTTTCAGAGC 2280
   AAGAGATTAC GCGCAGACCA AAACGATCTC AAGAAGATCA TCTTATTAAT CAGATAAAAT 2340
   ATTTCTAGAT TTCAGTGCAA TTTATCTCTT CAAATGTAGC ACCTGAAGTC AGCCCCATAC 2400
   GATATAAGTT GTTACTAGTG CTTGGATTCT CACCAATAAA AAACGCCCGG CGGCAACCGA 2460
   GCGTTCTGAA CAAATCCAGA TGGAGTTCTG AGGTCATTAC TGGATCTATC AACAGGAGTC 2520
   CAAGCGAGCT CTCGAACCCC AGAGTCCCGC TCAGAAGAAC TCGTCAAGAA GGCGATAGAA 2580
   GGCGATGCGC TGCGAATCGG GAGCGGCGAT ACCGTAAAGC ACGAGGAAGC GGTCAGCCCA 2640
   TTCGCCGCCA AGCTCTTCAG CAATATCACG GGTAGCCAAC GCTATGTCCT GATAGCGGTC 2700
   CGCCACACCC AGCCGGCCAC AGTCGATGAA TCCAGAAAAG CGGCCATTTT CCACCATGAT 2760
   ATTCGGCAAG CAGGCATCGC CATGGGTCAC GACGAGATCC TCGCCGTCGG GCATGCGCGC 2820
   CTTGAGCCTG GCGAACAGTT CGGCTGGCGC GAGCCCCTGA TGCTCTTCGT CCAGATCATC 2880
   CTGATCGACA AGACCGGCTT CCATCCGAGT ACGTGCTCGC TCGATGCGAT GTTTCGCTTG 2940
   GTGGTCGAAT GGGCAGGTAG CCGGATCAAG CGTATGCAGC CGCCGCATTG CATCAGCCAT 3000
   GATGGATACT TTCTCGGCAG GAGCAAGGTG AGATGACAGG AGATCCTGCC CCGGCACTTC 3060
   GCCCAATAGC AGCCAGTCCC TTCCCGCTTC AGTGACAACG TCGAGCACAG CTGCGCAAGG 3120
   AACGCCCGTC GTGGCCAGCC ACGATAGCCG CGCTGCCTCG TCCTGCAGTT CATTCAGGGC 3180
   ACCGGACAGG TCGGTCTTGA CAAAAAGAAC CGGGCGCCCC TGCGCTGACA GCCGGAACAC 3240
   GGCGGCATCA GAGCAGCCGA TTGTCTGTTG TGCCCAGTCA TAGCCGAATA GCCTCTCCAC 3300
   CCAAGCGGCC GGAGAACCTG CGTGCAATCC ATCTTGTTCA ATCATGCGAA ACGATCCTCA 3360
   TCCTGTCTCT TGATCAGATC TTGATCCCCT GCGCCATCAG ATCCTTGGCG GCAAGAAAGC 3420
   CATCCAGTTT ACTTTGCAGG GCTTCCCAAC CTTACCAGAG GGCGCCCCAG CTGGCAATTC 3480
   C                                                                3481
(2) INFORMATION ZU SEQ ID NO: 12:
   (i) SEQUENZ CHARAKTERISTIKA:
      (A) LÄNGE: 404 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 12:
   Met Lys Leu Pro Ile Tyr Leu Asp Tyr Ser Ala Thr Thr Pro Val Asp
     1               5                  10                  15
   Pro Arg Val Ala Glu Lys Met Met Gln Phe Met Thr Met Asp Gly Thr
                20                  25                  30
   Phe Gly Asn Pro Ala Ser Arg Ser His Arg Phe Gly Trp Gln Ala Glu
            35                  40                  45
   Glu Ala Val Asp Ile Ala Arg Asn Gln Ile Ala Asp Leu Val Gly Ala
        50                  55                  60
   Asp Pro Arg Glu Ile Val Phe Thr Ser Gly Ala Thr Glu Ser Asp Asn
    65                  70                  75                  80
   Leu Ala Ile Lys Gly Ala Ala Asn Phe Tyr Gln Lys Lys Gly Lys His
                    85                  90                  95
   Ile Ile Thr Ser Lys Thr Glu His Lys Ala Val Leu Asp Thr Cys Arg
               100                 105                 110
   Gln Leu Glu Arg Glu Gly Phe Glu Val Thr Tyr Leu Ala Pro Gln Arg
           115                 120                 125
   Asn Gly Ile Ile Asp Leu Lys Glu Leu Glu Ala Ala Met Arg Asp Asp
       130                 135                 140
   Thr Ile Leu Val Ser Ile Met His Val Asn Asn Glu Ile Gly Val Val
   145                 150                 155                 160
   Gln Asp Ile Ala Ala Ile Gly Glu Met Cys Arg Ala Arg Gly Ile Ile
                   165                 170                 175
   Tyr His Val Asp Ala Thr Gln Ser Val Gly Lys Leu Pro Ile Asp Leu
               180                 185                 190
   Ser Gln Leu Lys Val Asp Leu Met Ser Phe Ser Gly His Lys Ile Tyr
           195                 200                 205
   Gly Pro Lys Gly Ile Gly Ala Leu Tyr Val Arg Arg Lys Pro Arg Val
       210                 215                 220
   Arg Ile Glu Ala Gln Met His Gly Gly Gly His Glu Arg Gly Met Arg
   225                 230                 235                 240
   Ser Gly Thr Leu Pro Val His Gln Ile Val Gly Met Gly Glu Ala Tyr
                   245                 250                 255
   Arg Ile Ala Lys Glu Glu Met Ala Thr Glu Met Glu Arg Leu Arg Gly
               260                 265                 270
   Leu Arg Asn Arg Leu Trp Asn Gly Ile Lys Asp Ile Glu Glu Val Tyr
           275                 280                 285
   Leu Asn Gly Asp Leu Glu His Gly Ala Pro Asn Ile Leu Asn Val Ser
       290                 295                 300
   Phe Asn Tyr Val Glu Gly Glu Ser Leu Ile Met Ala Leu Lys Asp Leu
   305                 310                 315                 320
   Ala Val Ser Ser Gly Ser Ala Cys Thr Ser Ala Ser Leu Glu Pro Ser
                   325                 330                 335
   Tyr Val Leu Arg Ala Leu Gly Leu Asn Asp Glu Leu Ala His Ser Ser
               340                 345                 350
   Ile Arg Phe Ser Leu Gly Arg Phe Thr Thr Glu Glu Glu Ile Asp Tyr
           355                 360                 365
   Thr Ile Glu Leu Val Arg Lys Ser Ile Gly Arg Leu Arg Asp Leu Ser
       370                 375                 380
   Pro Leu Trp Glu Met Tyr Lys Gln Gly Val Asp Leu Asn Ser Ile Glu
   385                 390                 395                 400
   Trp Ala His His

## Beschreibung

Die Erfindung betrifft ein Genkonstrukt enthaltend ein Biotingen mit der Sequenz SEQ ID No. 1 oder SEQ ID No. 3, Organismen, die dieses Genkonstrukt enthalten, die Verwendung dieser Sequenzen oder des Genkonstrukts zur Herstellung von Biotin, sowie ein Verfahren zur Herstellung von Biotin.

Als Coenzym spielt Biotin (Vitamin H) eine essentielle Rolle bei enzymkatalysierten Carboxylierungs- und Decarboxylierungsreaktionen. Biotin ist damit ein essentieller Faktor in lebenden Zellen. Biotin muß von fast allen Tieren und einigen Mikroorganismen von außen aufgenommen werden, da sie Biotin nicht selber synthetisieren können. Es ist damit für diese Organismen ein essentielles Vitamin. Bakterien, Hefen und Pflanzen hingegen können Biotin aus Vorstufen selbst synthetisieren (Brown et al. Biotechnol. Genet. Eng. Rev. 9, 1991: 295 - 326, DeMoll, E., Escherichia coli and Salmonella, eds. Neidhardt, F. C. et al. ASM Press, Washington DC, USA, 1996: 704 - 708, ISBN 1-55581-084-5).

Die Biotinsynthese wurde in bakteriellen Organismen speziell im gramnegativen Bakterium Escherichia coli und im grampositiven Bakterium Bacillus sphaericus untersucht (Brown et al. Biotechnol. Genet. Eng. Rev. 9, 1991: 295 - 326). Als erstes bisher bekanntes Intermediat in E. coli wird Pimelyl-CoA (PmCoA) angesehen, das aus der Fettsäuresynthese stammt (DeMoll, E., Escherichia coli and Salmonella, eds. Neidhardt, F. C. et al. ASM Press, Washington DC, USA, 1996: 704 - 708, ISBN 1-55581-084-5 1996). Der Syntheseweg dieser Biotin-Vorstufe in E. coli ist bisher weitgehend unbekannt (Ifuku 1993, Lemoine 1996). Es wurden mit bioC und bioH zwei Gene identifiziert, deren korrespondierende Proteine für die Synthese von Pm-CoA verantwortlich sind. Die enzymatische Funktion der Genprodukte BioH und BioC ist bisher nicht bekannt (Lemoine et al., Mol. Microbio. 19, 1996: 645 - 647, DeMoll, E., Escherichia coli and Salmonella, eds. Neidhardt, F. C. et al. ASM Press, Washington DC, USA, 1996: 704 - 708, ISBN 1-55581-084-5). Pm-CoA wird in vier weiteren enzymatischen Schritten zum Biotin umgewandelt. Ausgehend vom Pm-CoA findet zuerst die Kondensation mit Alanin zu 7-Keto-8-Amino-Pelargonsäure (KAPA) statt. Das Genprodukt für diese Umsetzung ist BioF (KAPA-Synthetase). KAPA wird mit dem Kosubstrat S-Adenosyl-Methionin durch BioA (DAPA-Aminotransferase) zu 7,8 Diamino-Pelargonsäure transaminiert. Der nächste Schritt führt nach einer ATP-abhängigen Carboxylierungsreaktion zum Dethiobiotin (DTB) und wird durch BioD (Dethiobiotin-Synthase) katalysiert. Im letzten Schritt findet die Umsetzung von DTB zu Biotin statt. Dieser Schritt wird durch BioB (Biotin-Synthetase) katalysiert. Die für die beschriebenen Proteine kodierenden Gene bioF, bioA, bioD, und bioB sind in E. coli auf einem bidirektionalem Operon kodiert. Dieses Operon liegt zwischen der λ-attachment-site und dem uvrB Gen Locus bei ca. 17 Minuten auf dem E. coli Chromosom (Berlyn et al. 1996: 1715 - 1902). Auf diesem Operon sind zusätzlich noch zwei weitere Gene kodiert, von denen das eine, bioC, bereits beschriebene Funktionen in der Synthese von Pm-CoA hat, während einem offenen Leseraster hinter bioA bisher keine Funktion zugeordnet werden konnte (WO94/8023, Otsuka et al., J. Biol. Chem. 263, 1988: 19577 - 85). Hoch konservierte Homologe zu den E. coli Proteinen BioF, A, D, B wurden in B. sphaericus, B. subtilis, Syneccocystis sp. (Brown et al. Biotechnol. Genet. Eng. Rev. 9, 1991: 295 - 326, Bower et al., J. Bacteriol. 175, 1996: 4122 - 4130, Kaneko et al., DNA Res. 3, 3, 1996: 109 - 136), Archaebakterien wie Methanococcus janaschi, Hefen wie Saccharomyces cerevisiae (Zhang et al., Arch. Biochem. Biophys. 309, 1, 1994: 29 - 35) oder in Pflanzen wie Arabidopsis thaliana (Baldet et al., C. R. Acad. Sci. III, Sci. Vie. 319, 2, 1996: 99 - 106)) gefunden.

Die Synthese von Pm-CoA scheint in den beiden bisher untersuchten gram-positiven Mikroorganismen anders zu verlaufen als in E. coli. Es konnten keine Homologen von bioH und bioC gefunden werden (Brown et al. Biotechnol. Genet. Eng. Rev. 9, 1991: 295 - 326).

Biotin ist eine optisch aktive Substanz mit drei Chiralitätszentren. Es wird bisher wirtschaftlich nur über eine vielstufige, teure chemische Synthese hergestellt werden.

Alternativ zu dieser chemischen Synthese wurde eine Vielzahl von Versuchen unternommen, ein fermentatives Verfahren zur Herstellung von Biotin mit Mikroorganismen aufzubauen. Durch Klonierung des Biotin-Operons auf multi-copy-Plasmide konnte die Biotinsynthese in den mit diesen Genen transformierten Mikroorganismen erhöht werden. Eine weitere Erhöhung der Biotinsynthese wurde durch die Deregulierung der Biotingenexpression über die Selektion von birA-Mutanten erreicht (Pai C. H., J. Bacteriol. 112, 1972: 1280 - 1287). Die Kombination beider Ansätze, das heißt die Expression der Plasmid-kodierten Biosynthesegene in einem regulationsdefizienten Stamm (EP-B-0 236 429), brachte eine weitere Steigerung der Produktivität. Das Biotin-Operon kann dabei entweder unter Kontrolle seines nativen bidirektionalem Promotors verbleiben (EP-B-0 236 429), oder seine Gene können unter die Kontrolle eines extern regulierbaren Promotors gebracht werden (WO94/8023).

Durch die bisher verfolgten Ansätze zur fermentativen Herstellung von Biotin in E. coli konnte keine wirtschaftlich ausreichende Produktivität erreicht werden. Es zeigte sich, daß die Ausbeute in der fermentativen Herstellung von Biotin durch die unvollständige Umwandlung von DTB zu Biotin durch das BioB-Genprodukt (Biotinsynthase) verursacht wird. Zellen die Mutationen im bioB-Gen tragen, sind nicht in der Lage auf DTB zu wachsen und damit DTB in Biotin umzuwandeln. Der chemische und enzymatische Mechanismus der Umwandlung von DTB zu Biotin ist bisher nur unvollständig verstanden und aufgeklärt.

Durch intensive genetische Untersuchungen konnten bisher keine weiteren an der Reaktion beteiligten Proteine identifiziert werden. Eine Charakterisierung der Umwandlung von DTB zu Biotin wurde bisher ausschließlich in bakteriellen bzw. pflanzlichen Zellextrakten durchgeführt (WO94/8023, EP-B-0 449 724, Sanyal et al. Arch. Biochem. Biophys., Vol. 326, No. 1, 1996: 48 - 56 und Biochemistry 33, 1994: 3625 - 3631, Baldet et al. Europ. J. Biochem. 217, 1, 1993: 479 - 485, Mejean et al. Biochem. Biophys. Res. Commun., Vol. 217, No. 3, 1995: 1231 - 1237, Ohshiro et al., Biosci. Biotechnol. Biochem., 58, 9, 1994: 1738 - 1741).

Diese Untersuchungen haben gezeigt, daß niedermolekulare Faktoren wie S-Adenosylmethionin, NADPH, Cystein, Thiamin, Fe²⁺, Asparagin, Serin, Fruktose 1-6-bisphosphat die Synthese von Biotin stimulieren (Ohshiro et al., Biosci. Biotechnol. Biochem., 58, 9, 1994: 1738 - 1741, Birch et al., J. Biol. Chem. 270, 32, 1995: 19158 - 19165, Ifuk et al., Biosci. Biotechnol. Biochem., 59, 2, 1995: 185 - 189). Neben diesen niedermolekularen Faktoren wurden weitere Proteine identifiziert, die die Synthese von Biotin aus DTB in Gegenwart von BioB stimulieren. Dabei handelt es sich um Flavodoxin und Flavodoxin-NADPH-Reduktase (Birch et al., J. Biol. Chem. 270, 32, 1995: 19158 - 19165, Ifuk et al., Biosci. Biotechnol. Biochem., 59, 2, 1995: 185 - 189, Sanyal et al., Arch. Biochem. Biophys. 326, 1, 1996: 48 - 56).

Die Biotin- und Liponsäuresynthese weisen eine große Homologie auf. In beiden Synthesewegen wird auf der letzten Synthesestufe zwischen nicht aktivierte C-Atome ein Schwefel bzw. zwei Schwefelatome inseriert. Die Synthese von Liponsäure ist bisher nur unzureichend charakterisiert (DeMoll, E., Escherichia coli and Salmonella, eds. Neidhardt, F. C. et al. ASM Press, Washington DC, USA, 1996: 704 - 708, ISBN 1-55581-084-5). Es wurden bisher nur zwei notwendige Gene in E. coli identifiziert: lipA und lipB. Beide Gene liegen in einem Operon, mit einem bisher nicht charakterisiertem Open Reading Frame (= ORF) zwischen beiden Genen. Ein weiters Gen lplA ist in der Lage Liponsäure über ein Lipoyl-AMP-Intermediat auf Lysin zu übertragen. Diese Reaktion hat somit Ähnlichkeit mit der Aktivität von birA. Zwischen LipA und BioB konnten durch Sequenzvergleiche homologe Bereiche in der Aminosäuresequenz identifiziert werden. Diese Umfassen unter anderem ein Cystein-Cluster. Es konnte gezeigt werden, daß LipA für den Einbau von zwei Schwefelatomen in die Liponsäure katalysiert (DeMoll, E., Escherichia coli and Salmonella, eds. Neidhardt, F. C. et al. ASM Press, Washington DC, USA, 1996: 704 - 708, ISBN 1-55581-084-5).

Über die Herkunft des Schwefels im Biotinmolekül existieren widersprüchliche Ergebnisse. Bei Untersuchungen der Biotinsynthese in Gesamtzellextrakten wurde gezeigt, daß in Gegenwart von ³⁵S-markiertem Cystein Radioaktivität in Biotin inkorporiert wurde; weder mit ³⁵S-markiertem Methionin noch mit S-AdenosylMethionin konnte ein Schwefel-Einbau ins Biotinmolekül nachgewiesen werden (Ifuku et al., Biosci. Biotechnol. Biochem. 59, 2, 1995: 184 - 189, Birch et al., J. Biol. Chem.270, 32, 1995: 19158 - 19165).

Im Gegensatz dazu stehen Untersuchungen mit gereinigtem BioB-Protein in Gegenwart von ³⁵S-markiertem Cystein und ohne Zugabe von Zellextrakten, bei denen zwar eine Biotin-Synthese aber kein Einbau von Radioaktivität in das Biotinmolekül beobachtet werden konnte (Sanyal et al., Arch. Biochem. Biophys. 326, 1, 1996: 48 - 56, Méjean et al., Biochem. Biophys. Res. Commun. 2127, 3, 1995: 1231 - 1237). Unter diesen Synthesebedingungen ohne Zellextraktzugabe war die gebildete Biotinmenge niedrig und entsprach maximal ca. 2 Mol Biotin/Mol BioB (Sanyal et al., Arch. Biochem. Biophys. 326, 1, 1996: 48 - 56) bzw. 0,1 Mol Biotin/Mol BioB (Méjean et al., Biochem. Biophys. Res. Commun. 2127, 3, 1995: 1231 - 1237). Nach diesen Untersuchungen kann ein Einbau von Schwefel in Biotin erfolgen, ohne das Cystein als Schwefeldonor verwendet wird. Diese Biotinbildung ohne externe S-Quelle könnte durch eine Übertragung von Schwefel aus dem in BioB nachgewiesenen 2Fe-2S Cluster erklärt werden. Die tatsächliche Schwefelquelle für die Biotinsynthese ist nach wie vor unklar. Damit konnte bisher in vitro keine echte katalytische Aktivität von BioB nachgewiesen werden.

Trotz dieser Vielzahl von Ansätzen reicht die Ausbeute an Biotin durch mikrobielle Fermentation bisher für eine industrielle Produktion nicht aus.

Aufgabe der vorliegenden Erfindung war es, ein technisches, fermentatives Verfahren zur Herstellung von Biotin zu entwickeln, daß eine möglichst optimale Umwandlung von Dethiobiotin in Biotin aufweist und damit eine verbesserte Biotinsynthese ermöglicht.

Diese Aufgabe wurde durch das erfindungsgemäße Verfahren zur Herstellung von Biotin, dadurch gekennzeichnet, daß man ein Biotingen mit der Sequenz SEQ ID No. 1 oder SEQ ID No. 3 in einem prokaryontischen oder eukaryontischen Wirtsorganismus, der in der Lage ist Biotin zu synthetisieren, exprimiert, diesen züchtet und das synthetisierte Biotin direkt, nach Abtrennung der Biomasse oder nach Reinigung des Biotins verwendet, gelöst.

Die im erfindungsgemäßen Verfahren verwendeten Biotingene mit den Sequenzen SEQ ID No. 1 bzw. SEQ ID No. 3 werden in der SwissProt-Datenbank unter den "Accession"-Nummern AE000364 und D90811 geführt. Die Sequenz D90811 wird außerdem von Aiba et al. in DNA Res. 3, 6, 1996: 363 - 377 beschrieben. Bei beiden Sequenzen wird in der Datenbank eine Homologie zum NifS-Protein vermerkt. Weitere Informationen zu diesen Sequenzen sind der Datenbank bzw. der Publikation nicht zu entnehmen.

Durch Expression der Sequenzen SEQ ID No. 1 und/oder SEQ ID No. 3 in einem prokaryontischen oder eukaryontischen Wirtsorganismus läßt sich die Produktivität der Biotinbiosynthese deutlich steigern. Durch die Expression der Gene wird die Synthese von Biotin aus Dethiobiotin um mindestens den Faktor 2 gegenüber der Kontrolle ohne diese Gene, bevorzugt um einen Faktor größer 3, gesteigert. Bevorzugt wird die Sequenz SEQ ID No. 1 verwendet.

Nach Isolierung und Sequenzierung sind die im erfindungsgemäßen Verfahren verwendeten Biotingene mit den Nukleotidsequenzen SEQ ID No. 1 und SEQ ID No. 3 erhältlich, die für die in SEQ ID NO: 2 und SEQ ID No. 4 angegebenen Aminosäuresequenzen kodieren.

Als prokaryontische Wirtsorganismen des erfindungsgemäßen Verfahrens kommen prinzipiell alle Biotin synthetisierenden gram-negativen oder gram-positiven Bakterien in Frage. Als gramnegative Bakterien seien beispielhaft Enterobacteriaceae wie die Gattungen Escherichia, Aerobacter, Enterobacter, Citrobacter, Shigella, Klebsiella, Serratia, Erwinia oder Salmonella, Pseudomonadaceae wie die Gattungen Pseudomonas, Xanthomonas, Burkholderia, Gluconobacter, Nitrosomonas, Nitrobacter, Methanomonas, Comamonas, Cellulomonas oder Acetobacter, Azotobacteraceae wie die Gattungen Azotobacter, Azomonas, Beijerinckia oder Derxia, Neisseriaceae wie die Gattungen Moraxella, Acinetobacter, Kingella, Neisseria oder Branhamella, die Rhizobiaceae wie die Gattungen Rhizobium oder Agrobacterium oder die gram-negativen Gattungen Zymomonas, Chromobacterium oder Flavobacterium, genannt. Als gram-positive Bakterien seien beispielhaft die Endosporen-bildenden gram-positiven aeroben oder anaeroben Bakterien wie die Gattungen Bacillus, Sporolactobacillus oder Clostridium, die coryneformen Bakterien wie die Gattungen Arthrobacter, Cellulomonas, Curtobacterium, Corynebacterium, Brevibacterium, Microbacterium oder Kurthia, die Actinomycetales wie die Gattungen Mycobacterium, Rhodococcus, Streptomyces oder Nocardia, die Lactobacillaceae wie die Gattungen Lactobacillus oder Lactococcus, die gram-positiven Kokken wie die Gattungen Micrococcus oder Staphylococcus, genannt.

Bevorzugt werden Bakterien der Gattungen Escherichia, Citrobacter, Serratia, Klebsiella, Salmonella, Pseudomonas, Comamonas, Acinetobacter, Azotobacter, Chromobacterium, Bacillus, Clostridium, Arthrobacter, Corynebacterium, Brevibacterium, Lactococcus, Lactobacillus, Streptomyces, Rhizobium, Agrobacterium oder Staphylococcus im erfindungsgemäßen Verfahren verwendet. Besonders bevorzugt werden Gattungen und Arten wie Escherichia coli, Citrobacter freundii, Serratia marcescens, Salmonella typhimurium, Pseudomonas mendocina, Pseudomonas aeruginosa, Pseudomonas mutabilis, Pseudomonas chlororaphis, Pseudomonas fluorescens, Comamonas acidovorans, Comamonas testosteroni, Acinetobacter calcoaceticus, Azotobacter vinelandii, Chromobacterium violaceum, Bacillus subtilis, Bacillus sphaericus, Bacillus stearothermophilus, Bacillus pumilus, Bacillus licheniformis, Bacillus amyloliquefaciens, Bacillus megaterium, Bacillus cereus, Bacillus thuringiensis, Arthrobacter citreus, Arthrobacter paraffineus, Corynebacterium glutamicum, Corynebacterium primorioxydans, Corynebacterium sp., Brevibacterium ketoglutamicum, Brevibacterium linens, Brevibacterium sp., Streptomyces lividans, Rhizobium leguminosarum oder Agrobacterium tumefaciens. Vorteilhafterweise werden Bakterien verwendet, die schon eine erhöhte natürliche Biotinproduktion besitzen.

Die taxonomische Stellung der aufgeführten Gattungen unterlag in den letzten Jahren einem starken Wandel und befindet sich noch immer im Fluß, da falsche Gattungs- und Artnamen korrigiert werden. Aufgrund dieser in der Vergangenheit häufig erforderlichen taxonomischen Umgruppierungen der genannten Gattungen innerhalb der bakteriellen Systematik sind auch andere als die oben genannten Familien, Gattungen und Arten für das erfindungsgemäße Verfahren geeignet.

Als eukaryontische Wirtsorganismen des erfindungsgemäßen Verfahrens kommen prinzipiell alle Biotin synthetisierenden Organismen in Frage wie Pilze, Hefen, Pflanzen oder pflanzliche Zellen. Als Hefen seien die Gattungen Rhodotorula, Yarrowia, Sporobolomyces, Saccharomyces oder Schizosaccharomyces bevorzugt genannt. Besonders bevorzugt sind die Gattungen und Arten Rhodotorula rubra, Rhodotorula glutinis, Rhodotorula graminis, Yarrowia lipolytica, Sporobolomyces salmonicolor, Sporobolomyces shibatanus oder Saccharomyces cerevisiae.

Als Wirtsorganismus können prinzipiell alle Pflanzen verwendet werden, bevorzugt werden Pflanzen, die in der Tierernährung oder in der humanen Ernährung eine Rolle spielen wie Mais, Weizen, Gerste, Roggen, Kartoffeln, Erbsen, Bohnen, Sonnenblumen, Palmen, Hirse, Sesam, Kopra oder Raps. Auch Pflanzen wie Arabidopsis thaliana oder Lavendula vera sind geeignet. Besonders bevorzugt werden pflanzliche Zellkulturen, Protoplasten aus Pflanzen oder Kaluskulturen.

Vorteilhafterweise werden im erfindungsgemäßen Verfahren Mikroorganismen wie Bakterien, Pilze, Hefen oder pflanzliche Zellen verwendet, die in der Lage sind Biotin in das Anzuchtsmedium auszuscheiden und die gegebenenfalls zusätzlich schon eine erhöhte natürliche Biotinsynthese haben. Vorteilhafterweise können diese Organismen noch bezüglich der Regulation ihrer Biotinbiosynthese defekt sein, das heißt es findet keine oder nur eine sehr verringerte Regulation der Synthese statt. Dieser Regulationsdefekt hat zur Folge, daß diese Organismen schon eine wesentlich höhere Biotinproduktivität besitzen. Ein solcher Regulationsdefekt ist beispielsweise von Escherichia coli als birA-Defektmutanten bekannt und sollte vorzugsweise in Form eines durch äußere Einflüsse induzierbaren Defektes, beispielsweise temperaturinduzierbar, in den Zellen vorhanden sein. Es können im Prinzip auch Organismen verwendet werden, die keine natürliche Biotinproduktion aufweisen, nachdem sie mit den Biotingenen transformiert wurden.

Um die Biotinproduktivität insgesamt weiter zu steigern sollten die Organismen im erfindungsgemäßen Verfahren vorteilhafterweise zusätzlich mindesten ein weiteres Biotingen ausgewählt aus der Gruppe bioA, bioB, bioF, bioC, bioD, bioH, bioP, bioW, bioX, bioY oder bioR enthalten. Dieses zusätzliche Gen oder diese zusätzlichen Gene können in ein oder mehreren Kopien in der Zelle vorhanden sein. Sie können auf dem gleichen Vektor wie die Sequenzen SEQ ID No. 1 und/oder SEQ ID No. 3 lokalisiert sein oder auf getrennten Vektoren oder aber chromosomal integriert worden sein. Auch die Sequenzen SEQ ID No. 1 und/oder SEQ ID No. 3 können ins Genom inseriert werden.

Unter dem erfindungsgemäßen Genkonstrukt sind die Biotingensequenzen SEQ ID No. 1 und SEQ ID No. 3 zu verstehen, die mit einem oder mehreren Regulationssignalen zur Erhöhung der Genexpression funktionell verknüpft wurden. Zusätzlich zu diesen neuen Regulationssequenzen kann die natürliche Regulation dieser Sequenzen vor den eigentlichen Strukturgenen noch vorhanden sein und gegebenenfalls genetisch verändert worden sein, so daß die natürliche Regulation ausgeschaltet und die Expression der Gene erhöht wurde. Das Genkonstrukt kann aber auch einfacher aufgebaut sein, das heißt es wurden keine zusätzlichen Regulationssignale vor die Sequenzen SEQ ID No. 1 und/oder SEQ ID No. 3 inseriert und der natürliche Promotor mit seiner Regulation wurde nicht entfernt. Stattdessen wurde die natürliche Regulationssequenz so mutiert, daß keine Regulation durch Biotin mehr erfolgt und die Genexpression gesteigert wird. Auch am 3'-Ende der DNA-Sequenzen können zusätzliche vorteilhafte regulatorische Elemente inseriert werden. Die Biotingene mit den Sequenzen SEQ ID No. 1 und/oder SEQ ID No. 3 können in einer oder mehreren Kopien im Genkonstrukt enthalten sein.

Vorteilhafte Regulationssequenzen für das erfindungsgemäße Verfahren sind beispielsweise in Promotoren wie cos-, tac-, trp-, tet-, trp-tet-, lpp-, lac-, lpp-lac-, lacI^{q-}, T7-, T5-, T3-, gal-, trc-, ara-, SP6-, λ-P_{R}- oder im λ-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefepromotoren ADC1, MFα, AC, P-60, CYC1, GAPDH oder in den Pflanzenpromotoren CaMV/35S, SSU, OCS, lib4, usp, STLS1, B33, nos oder im Ubiquitin- oder Phaseolin-Promotor enthalten.

Prinzipiell können alle natürlichen Promotoren mit ihren Regulationssequenzen wie die oben genannten für das erfindungsgemäße Verfahren verwendet werden. Darüberhinaus können auch synthetische Promotoren vorteilhaft verwendet werden.

Im Genkonstrukt sind weitere Biotingene ausgewählt aus der Gruppe bioA, bioB, bioF, bioC, bioD, bioH, bioP, bioW, bioX, bioY oder bioR in einer oder mehreren Kopien enthalten, ausgenommen Genkonstrukte, die ein nifS-Gen aus Klebsiella pneumoniae und ein bioB-Gen aus E.-coli enthalten, die einen eigenen Promotor haben können oder aber unter der Regulation des Promotors der Sequenzen SEQ ID No. 1 oder SEQ ID No. 3 liegen können.

Das Genkonstrukt wird zur Expression in den oben genannten Wirtsorganismus vorteilhafterweise in einen wirtsspezifischen Vektor inseriert, der eine optimale Expression der Gene im Wirt ermöglicht. Geeignete Vektoren sind beispielsweise in E. coli pLG338, pACYC184, pBR322, pUC18, pUC19, pKC30, pRep4, pBS1, pHS2, pPLc236, pMBL24, pLG200, pUR290, pIN-III¹¹³-B1, λgt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen YEp6, YEp13 oder pEM-BLYe23 oder in Pflanzen pLGV23, pGHlac⁺, pBIN19, pAK2004 oder pDH51. Die genannten Vektoren stellen eine kleine Auswahl der möglichen Vektoren dar. Weitere Vektoren sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

Unter Expressionssysteme sind die Kombination aus den oben beispielhaft genannten Wirtsorganismen und den zu den Organismen passenden Vektoren wie Plasmide, Viren oder Phagen wie das T7 RNA Polymerase/Promoter System oder Vektoren mit regulatorischen Sequenzen für den Phagen λ zu verstehen.

Bevorzugt sind unter dem Begriff Expressionssysteme die Kombination aus Escherichia coli und seinen Plasmiden und Phagen und den dazugehörenden Promotoren sowie Bacillus und seine Plasmide und Promotoren zu verstehen.

Für die vorteilhafte erfindungsgemäße Expression der SEQ ID No. 1 und SEQ ID No. 3 sind außerdem weitere 3' und/oder 5' Terminale regulatorische Sequenzen geeignet.

Diese regulatorischen Sequenzen sollen die gezielte Expression der Biotingene und der Proteinexpression ermöglichen. Dies kann beispielsweise je nach Wirtsorganismus bedeuten, daß das Gen erst nach Induktion exprimiert oder überexprimiert wird, oder daß es sofort exprimiert und/oder überexprimiert wird.

Die regulatorischen Sequenzen bzw. Faktoren können dabei vorzugsweise die Biotingenexpression positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Unter "Enhancer" sind beispielsweise DNA-Sequenzen zu verstehen, die über eine verbesserte Wechselwirkung zwischen RNA-Polymerase und DNA eine erhöhte Biotingenexpression bewirken.

Eine Steigerung der von den Sequenzen SEQ ID No. 1 oder SEQ ID No. 3 abgeleiteten Proteinen und ihrer Enzymaktivität läßt sich zum Beispiel gegenüber den Ausgangsenzymen durch Veränderung der entsprechenden Gensequenzen oder der Sequenzen seiner Homologen durch klassische Mutagenese wie UV-Bestrahlung oder Behandlung mit chemischen Mutagentien und/oder durch gezielte Mutagenese wie site directed mutagenesis, Deletion(en), Insertion(en) und/oder Substitution(en) erzielen. Auch kann eine erhöhte Enzymaktivität neben der beschriebenen Genamplifikation durch Ausschaltung von Faktoren, die die Enzymbiosynthese reprimieren und/oder durch Synthese aktiver statt inaktiver Enzyme erreicht werden.

Durch das erfindungsgemäße Verfahren wird die Umwandlung von DTB in Biotin und damit die Biotinproduktivität insgesamt über die in die Organismen über Vektoren und/oder chromosomal klonierten Biotingene mit den Sequenzen SEQ ID No. 1 oder SEQ ID No. 3 vorteilhaft gesteigert.

Im erfindungsgemäßen Verfahren werden die SEQ ID No. 1 und/oder SEQ ID No. 3 enthaltenen Mikroorganismen in einem Medium, das das Wachstum dieser Organismen ermöglicht angezüchtet. Dieses Medium kann ein synthetisches oder ein natürliches Medium sein. Je nach Organismus werden dem Fachmann bekannte Medien verwendet. Für das Wachstum der Mikroorganismen enthalten die verwendeten Medien eine Kohlenstoffquelle, eine Stickstoffquelle, anorganische Salze und gegebenenfalls geringe Mengen an Vitamine und Spurenelemente.

Vorteilhafte Kohlenstoffquellen sind beispielsweise Zucker wie Mono-, Di- oder Polysaccharide wie Glucose, Fructose, Mannose, Xylose, Galactose, Ribose, Sorbose, Ribulose, Lactose, Maltose, Saccharose, Raffinose, Stärke oder Cellulose, komplexe Zuckerquellen wie Melasse, Zuckerphosphate wie Fructose-1,6-bis-phosphat, Zuckeralkohole wie Mannit, Polyole wie Glycerin, Alkohole wie Methanol oder Ethanol, Carbonsäuren wie Citronensäure, Milchsäure oder Essigsäure, Fette wie Sojaöl oder Rapsöl, Aminosäuren wie Glutaminsäure oder Asparaginsäure oder Aminozucker, die auch gleichzeitig als Stickstoffquelle verwendet werden können.

Vorteilhafte Stickstoffquellen sind organische oder anorganische Stickstoffverbindungen oder Materialien, die diese Verbindungen enthalten. Beispiele sind Ammoniumsalze wie NH₄Cl oder (NH₄)₂SO₄, Nitrate, Harnstoff, oder komplexe Stickstoffquellen wie Maisquellwasser, Bierhefeautolysat, Sojabohnenmehl, Weizengluten, Hefeextrakt, Fleischextrakt, Caseinhydrolysat, Hefe oder Kartoffelprotein, die häufig auch gleichzeitig als Stickstoffquelle dienen können.

Beispiele für anorganische Salze sind die Salze von Calcium, Magnesium, Natrium, Mangan, Kalium, Zink, Kupfer und Eisen. Als Anion dieser Salze sind besonders das Chlor-, Sulfat- und Phosphation zu nennen. Ein wichtiger Faktor zur Steigerung der Produktivität im erfindungsgemäßen Verfahren ist der Zusatz von Fe²⁺⁻ oder Fe³⁺-Salzen und/oder Kaliumsalzen zum Produktionsmedium.

Gegebenenfalls werden dem Nährmedium weitere Wachstumsfaktoren zugesetzt, wie beispielsweise Vitamine oder Wachstumsförderer wie Riboflavin, Thiamin, Folsäure, Nicotinsäure, Pantothenat oder Pyridoxin, Aminosäuren wie Alanin, Cystein, Asparagin, Asparaginsäure, Glutamin, Serin, Methonin oder Lysin, Carbonsäuren wie Citronensäure, Ameisensäure, Pimelinsäure oder Milchsäure, oder Substanzen wie Dithiothreitol.

Zur Stabilisierung der Vektoren mit den Biotingenen in den Zellen können gegebenenfalls Antibiotika dem Medium zugesetzt werden.

Das Mischungsverhältnis der genannten Nährstoffe hängt von der Art der Fermentation ab und wird im Einzelfall festgelegt. Die Mediumkomponenten können alle zu Beginn der Fermentation vorgelegt werden, nachdem sie falls erforderlich getrennt sterilisiert oder gemeinsam sterilisiert wurden, oder aber je nach Bedarf während der Fermentation nachgegeben werden.

Die Züchtungsbedingungen werden so festgelegt, daß die Organismen optimal wachsen und daß die bestmöglichen Ausbeuten erreicht werden. Bevorzugte Züchtungstemperaturen liegen bei 15 °C bis 40 °C. Besonders vorteilhaft sind Temperaturen zwischen 25 °C und 37 °C. Vorzugsweise wird der pH-Wert in einem Bereich von 3 bis 9 festgehalten. Besonders vorteilhaft sind pH-Werte zwischen 5 und 8. Im allgemeinen ist eine Inkubationsdauer von 8 bis 240 Stunden bevorzugt von 8 bis 120 Stunden ausreichend. Innerhalb diser Zeit reichert sich die maximale Menge an Biotin im Medium an und/oder ist nach Aufschluß der Zellen verfügbar.

Das erfindungsgemäße Verfahren zur Herstellung von Biotin kann kontinuierlich oder batch-weise durchgeführt werden. Werden aus den mit den Biotingenen transformierten Pflanzenzellen ganze Pflanzen regeneriert, so können diese nach dem erfindungsgemäßen Verfahren ganz normal angezüchtet und vermehrt werden.

### Beispiele:

Ausgehend von der Überlegung, daß an der Umsetzung von DTB zu Biotin außer BioB und den bekannten weiteren Kofaktoren möglicherweise ein Fe-S-Cluster-regenerierendes Enzym beteiligt sein könnte, wurde der Versuch unternommen ein solches hypotetisches Gen zu identifizieren und zu klonieren.

NifS-Gene sind in der Lage Schwefelatome in einem Fe-S-Cluster von Proteinen, die an der Stickstofffixierung beteiligt sind, zu regenerieren (Zheng et al., Proc. Natl. Acad. Sci. USA 90, 1993: 2754 - 2758 und Biochemistry 33, 1994: 4714 - 4720). Durch Vergleich aller bekannten Proteine der NifS-Klasse in den Datenbanken Swiss-Prot und PIR mit dem Programmpaket Lasergene (DNA-Star Inc.) unter dem Megalign Modus, konnte ein streng konservierter Bereich dieser Proteine mit der Aminosäuresequenz HK(I,L)xGPxG (x entspricht Aminosäuren mit geringer bzw. nicht erhaltener Konservierung) identifiziert werden. Die Tatsache der vollständigen Konservierung dieser Sequenz deutet auf eine wichtige Rolle dieser Aminosäuren (= As) in der Funktion dieser Proteinen hin. Diese konservierten Aminosäuren werden im folgenden als Motiv I bezeichnet.

Das derart beschriebene As-Motiv diente als Vergleichsequenz für eine weitere Analyse der Datenbank Swiss-Prot/PIR Release 93 bzw. 94 nach Proteinen bzw. ORF's (= open reading frame) zu suchen, in denen diese NifS-Funktionssequenz vollständig konserviert ist. Als Programm für die Sequenzanalyse wurde das Programm Geneman des Pakets DNA-Star eingesetzt. Die Analyseparameter wurden wie folgt fixiert: Menue "consensus sequence" mit einer 80% Konservierung des Motivs.

Als Ergebnis dieser Recherche wurde gefunden, daß neben bereits als NifS-homolge Proteine identifizierte Proteine weitere Proteine/ bzw. ORF's existieren, die dieses Sequenzmotiv aufweisen. Unter den in der Datenbank enthaltenen weiteren Sequenzen konnte ein offenes Leseraster aus E. coli mit signifikanter Konservierung der Konsensus-Sequenz identifiziert werden, das wie die eigenen Arbeiten zeigten an der Biotinsynthese beteiligt ist. Dieses offene Leseraster mit der Bezeichnung ECU29581_24 (= SEQ ID No. 1 = ORF401) kodiert für ein hypothetisches von dieser Sequenz abgeleitetes Protein von 401 As. Es zeigte sich, daß diese Sequenz im Rahmen der E. coli Genomsequenzierung durch F. Blattner und Mitarbeitern sequenziert worden war (DNA-Research 1996), ohne daß dessen Funktion erkannt worden war. Diese Sequenz (SEQ ID No. 1) wird im Nachfolgenden als BioS1 bezeichnet.

Vergleicht man die Proteinsequenz von BioS1 mit der von NifS aus A. vinelandii (Programm: DNA-Star-"Megalign" im Modus: paarweises Alignement nach Lipman-Pearson, Analyseparameter: k-tuple 2, gap-penalty 4, gap-length-penalty 12) zeigt sich, daß ECU29581_24 auch in anderen Bereichen der Sequenz über einen Sequenzbereich von 218 As eine Homologie von 27,6% zu NifS aus A. vinelandii aufweist. Gegenüber dem als NifS identifiziertem Protein aus Rhodococcus capsulatus beträgt die Homologie 25,3 % über einen Bereich von 376 As.

In der Datenbank Swiss-Prot/PIR konnten weitere Sequenzen identifiziert werden, die Homologien zu ECU29581_24 aufweisen (Programm Geneman/ Modus sequence similarity; Einstellungen default). Die höchste gefundene Sequenzähnlichkeit zu ECU29581_24 zeigt ein translatierter ORF (= open reading frame) aus H. influenza (Datenbankbezeichnung HIU00082_62). Zwischen BioS1 und HIU00082_62 wurde eine Sequenzhomolgie von 45,5% über die gesamte Länge beider Proteine gefunden. Die Sequenzähnlichkeit bzw. die Homologie beider Proteine ist damit signifikant höher, als zwischen ECU29581_24 (= BioS1) und NifS aus R. capsulatus bzw. A. vinelandii. Es handelt sich damit vermutlich um das H. influenza Homologe zu BioS1.

Von Fleischmann et al. (Science. 269, 1995: 496-512) war neben dem ORF HIU00082_62 ein weiterer ORF (HIU00072_10) in H. influenza aufgrund seiner Sequenzähnlichkeit zu NifS gefunden worden.

Aufgrund dieser Beschreibung von Fleischmann et al. wurde gefolgert, daß neben bioS1 auch in E. coli noch ein weiteres NifS-ähnliches Gen existiert. Dieses hypothetische Gen wurde bioS2 genannt.

### 1. Konstruktion der Vektoren pHS1und pHS2:

Die Plasmide pHS1 und pHS2 bestehen aus verschiedenen sog. Kassetten, die einen Replikationsursprung, eine Resistenzkassette, einen Promotor, eine Klonierungsstelle, und Terminatoren tragen. Die Plasmide wurden aus unterschiedlichen DNA-Fragmenten zusammengesetzt. Die dafür notwendigen DNA-Fragmente wurden durch PCR-Reaktionen mit unterschiedlichen Plasmiden als Matrizen hergestellt.

### a.) Herstellung der Kassette mit einem Replikationsursprung:

Um den Replikationsursprung aus einem P15A-Replikon-enthaltenden Plasmid als klonierbare Kassette bereitzustellen, wurde durch eine PCR Reaktion mit dem Plasmid pRep4 (Quiagen) mit den Oligonukleotiden P15A,1 (5'-GGCCCCTAGGGGATATATTCCGCTTCCTCGC-3') und P15A,2 (5'-GGCCACTAGTAACAACTTATATCGTATGGGG-3') ein DNA-Fragment von 919 Basen Länge aus dem Plasmid isoliert. Das Fragment wurde mit den Restriktionsenzymen AvrII und SpeI in einem geeigneten Puffer geschnitten.

### PCR-Bedingungen:

Es wurden 2,5 U Taq-Polymerase sowie 15 pMol der Oligonukleotide in 100 µl Ansatzlösung zur Isolierung der Replikationskassette aus dem Plasmid pRep4 verwendet. Die Oligonukleotide wurden bei 50°C angelagert. Die Elongation erfolgte bei 72 °C für 1 min. über 30 Zyklen.

### b.) Herstellung der Kanamyzin-Resistenzkassette:

Um eine Kanamyzin-Resistenzkassette als klonierbare Kassette bereitzustellen wurde durch eine PCR Reaktion aus einem die Kanmyzin-Resistenzkassette enthaltenden Plasmid (pRep4, Quiagen) mit den Oligonukleotiden Kan-R,1 (5'-GGCCGAGCTCTCGAACCCCA-GAGTCCCGCT-3') und Kan-R,2 (5'-GGCCGACGTCGGAATTGCCAGCTGGGGCGC-3') ein DNA-Fragment von 952 Basen Länge isoliert. Das Fragment wurde mit AatII und SacI in einem geeigneten Puffer geschnitten.

### PCR-Bedingungen:

Es wurden 2,5 U Taq-Polymerase sowie 15 pMol der Oligonukleotide in 100 µl Ansatzlösung zur Isolierung der Kanamyzin-Resistenzkassette aus dem Plasmid pRep4 verwendet. Die Oligonukleotide wurden bei 50°C angelagert. Die Elongation erfolgte bei 72 °C für 1 min. über 30 Zyklen.

### c.) Herstellung der Terminationsbereiche:

* Um den Terminator T0 aus dem Phagen Lambda als klonierbare Kassette bereitzustellen wurde durch eine PCR Reaktion mit dem Plasmid pDS12-luzi (Schroder H. et al., EMBO Journal. 12, 11, 1993: 4137 - 4144) als Matrize mit den Oligonukleotiden T0,1 (5'-GGCCGAGCTCGCTTGGACTCCTGTTGATAG-3') und T0, 2 (5'-GGCCACTAGTGCTTGGATTCTCACCAATAAAAAACGCCC-3') ein DNA-Fragment von 120 Basen Länge isoliert. Das Fragment wurde durch die Enzyme SpeI und SacI in einem geeigneten Puffer geschnitten.
   Matrize für T0: pDS12-luzi
   Es wurden 2,5 U Taq-Polymerase sowie 15 pMol der Oligonukleotide in 100 µl Ansatzlösung zur Isolierung des Terminationsbereiches aus dem Plasmid pDS12-luzi verwendet. Die Oligonukleotide wurden bei 50 °C angelagert. Die Elongation erfolgte bei 72 °C für 0,5 min. über 30 Zyklen. Anschließend wurde ein 120 bp großes Fragment isoliert und aufgereinigt. Dieses wurde mit je 20 U SpeI und SacI verdaut.
* Um den Terminator T1 aus dem rrnB-Operon als klonierbare Kassette bereitzustellen, wurde durch eine PCR Reaktion mit dem Plasmid pDS12-luzi (Schroder et al. siehe oben) als Matrize und mit Hilfe der Oligonukleotide T1,1 (5'-GGCCCCTAG-GTCTAGGGCGGCGGATTTGTCC-3') und T1,2 (5'-GGCCTCTAGAGGCATCAAA-TAAAACGAAAGGC-3') ein DNA-Fragment von 120 bp Länge isoliert. Das Fragment wurde durch die Enzyme XbaI und AvrII in einem geeigneten Puffer geschnitten.
   Matrize für T1: pDS12-luzi
   Es wurden 2,5 U Taq-Polymerase sowie 15 pMol der Oligonukleotide in 100 µl Ansatzlösung zur Isolierung des Terminationsbereiches aus dem Plasmid pDS12-luzi verwendet. Die Oligonukleotide wurden bei 50 °C angelagert. Die Elongation erfolgte bei 72 °C für 0,5 min. über 30 Zyklen. Anschließend wurde ein 120 bp großes Fragment isoliert und aufgereinigt. Dieses wurde mit je 20 U XbaI und AvrII verdaut.

### d.) Herstellung der Promotoren für pHS1 und pHS2:

Die Oligonukleotide PPHS1,1
(5'-TCGAGATAGCATTTTTATCCATAAGATTAGCCGATCCTAAGGTTTACAATTGTGAGCGCTC ACAATTATGATAGATTCAATTGTGAGCGGATAACAATTTCACACACGCTAGCGGTAC-3') und PPHS1,2
(5'-CGCTAGCGTGTGTGAAATTGTTATCCGCTCACAATTGAATCTATCATAATTGTGAGCGCTC ACAATTGTAAACCTTAGGATCGGCTAATCTTATGGATAAAAATGCTATC-3') bzw. PPHS2,1
(5'-AATTCTCCCTATCAGTGATAGAGATTGACATCCCTATCAGTGATAGAGATACTGAGACATC ACCAGGACGCACTGACCG-3'9 und PPHS2,2
(5'-AATTCGGTCAGTGCGTCCTGGTGATGTCTCAGTATCTCTATCACTGATAGGGATGTCAATC TCTATCACTGATAGGGAGG-3') wurden durch chemische Synthese hergestellt. Die Oligonukleotide PPHS1,1 und PPHS1,2 sowie PPHS2,1 und PPHS2,2 wurden jeweils in einer Konzentration von 1µg/µl vermischt, 5 min bei 95 °C inkubiert und dann langsam abgekühlt. Die aneinandergelagerten Oligonukleotide wurden in einer Konzentration von 10 ng/µl in der Ligation eingesetzt. Die Oligonucleotide PPHS1,1 und PPHS1,2 bildeten den Promotor für das Plasmid pHS1 und die Oligonucleotide PPHS2,1 und PPHS2,2 den für das Plasmid pHS2.

### e.) Herstellung der Klonierungsstelle:

Zur Konstruktion der Klonierungsstelle wurden die beiden Oligonukleotide PMCS1,1
(5'-GTACCGGGCCCCCCCTCGAGGTCGACGGTATCGATAAGCTTGATATCGAATTCCTGCAGCC CGGGGGATCCCATGGTA-3') und PMCS1,2
(5'-ACGCGTACCATGGGATCCCCCGGGCTGCAGGAATTCGATATCAAGCTTATCGATACCGTCG ACCTCGAGGGGGGGCCCGGTACC-3') synthetisiert. Die beiden Oligonukleotide wurden in einer Konzentration von 1µg/µl vermischt, 5 min bei 95 °C inkubiert und dann langsam abgekühlt. Die aneinandergelagerten Oligonukleotide wurden in einer Konzentration von 10 ng/µl in der Ligation eingesetzt.

### f.) Klonierungsablauf von pHS1 und pHS2

Ausgehend von pDS12 luci wurde die ampR-Kassette des Plasmides durch einen SacI/AatII-Verdau herausgeschnitten und durch ein entsprechendes SacI/AatII-Fragment ersetzt, das die Kanamyzin-Resitenzkassette enthält. Nach Transformation und Isolierung positiver Klone wurde der erhaltene Vektor SpeI/SacI verdaut und der durch PCR-amplifizierte Terminator T0 als SpeI/SacI-Fragment stromabwärts der Kanamyzin-Resistenzkassette eingesetzt. Nach Transformation und Isolierung positiver Klone wurde der erhaltene Vektor wurde mit XbaI/AvrII verdaut und der durch PCR-amplifizierte Terminator T1 als XbaI/AvrII-Fragment eingesetzt. Der erhaltene Vektor wurde mit XhoI/EcoRI verdaut und mit den jeweils aneinandergelagerten Promotoroligonukleotiden (PPHS1,1 und PPHS1,2 bzw. PPHS2,1 und PPHS2,2) ligiert. Der entstandene Vektor wurde XbaI verdaut und mit Hilfe von Klenow-Fragment aufgefüllt, dann KpnI verdaut und die Vektorbande ohne das Luziferase-Fragment isoliert. Zwei weitere aneinandergelagerte Oligonukleotide (PMCS1,1 PMCS1,2), die die Klonierungsstelle enthalten, wurden mit den so verdauten Vektoren ligiert.

### 2. Klonierung von bioS1 (ECU29581_24, SEQ ID No. 1):

Das Gen, das für BioS1 kodiert, wurde aus dem Chromosom von E. coli durch eine PCR-Reaktion amplifiziert, mit otimierten Translationssignalen versehen und in einen Vektor kloniert, der eine Überexpression des Gens in E. coli Stämmen ermöglicht.

### a.) Entwicklung von Oligonukleotiden zur Amplifizierung des bioS1 Gens aus dem E. coli Chromosom:

BioS1 soll als Expressionskassette bestehend aus einer ribosomalen Bindungstelle, dem Startkodon der kodierenden Sequenz und dem Stopkodon zwischen zwei Erkennungstellen für Restriktionsenzyme amplifiziert werden. Für beide Restriktionsschnittstellen wurde die Erkennungssequenz von MluI gewählt. Das bioS1 Gen wurde mit Hilfe der Oligonucleotide PbioS1,1 (5'-CGCACGCGTGAGGAGTACCAT-GAACGT-3') und PbioS1,2 (5'-CGCACGCGTTTAATCCACCAATAATT-3') kloniert.

### Durchführung der PCR:

### Bedingungen:

Als Matrize wurden 0,5µg chromosomale DNA von E. coli W3110 verwendet. Die Oligonukleotide PbioS1,1 und PbioS1,2 wurden in einer Konzentration von je 15 pMol eingesetzt. Die Konzentration an dNTP's betrug 200 µM. Als Polymerase wurden 2,5 U Pwo DNA-Polymerase (Boehringer Mannheim) im Reaktionspuffer des Herstellers eingesetzt. Das Volumen der PCR-Reaktion betrug 100µl.

### Amplifikationsbedingungen:

Die Denaturierung der DNA erfolgte für 2 min bei 94 °C. Anschließend wurden die Oligonukleotide für 30 sec bei 55 °C angelagert. Die Elongation erfolgte für 45 sec bei 72 °C. Die PCR-Reaktion wurde über 30 Zyklen durchgeführt.

Das erhaltene DNA-Produkt mit einer Größe von 1200 bp wurde aufgereinigt und durch MluI im geeigneten Puffer verdaut.

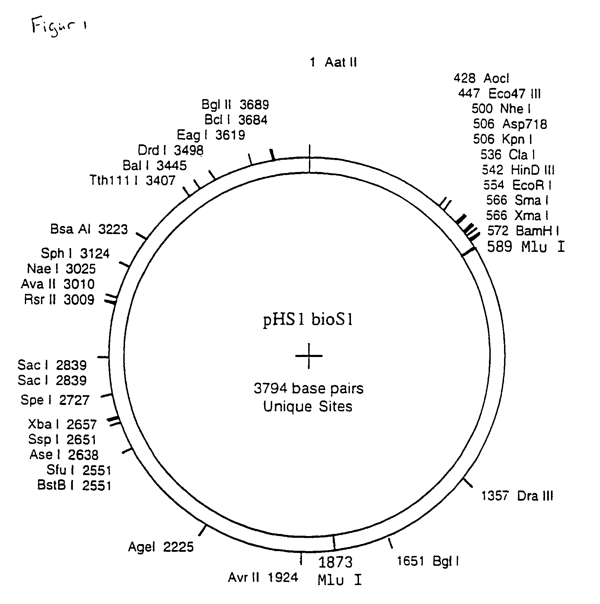

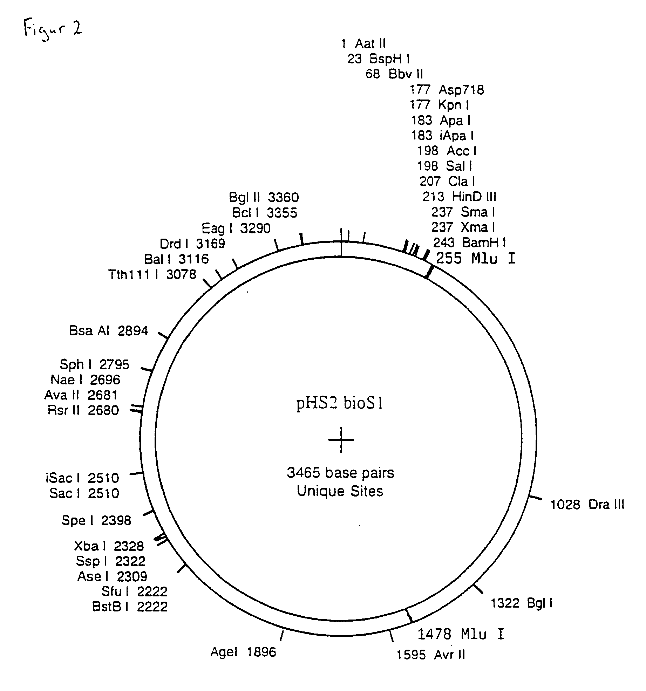

5µg des Vektors pHS1 wurden duch MluI verdaut und durch Shrimp Alkalische Phosphatase (SAP) (Boehringer Mannheim) dephosphorylliert. Nach Denaturierung der SAP wurden Vektor und Fragment in einem molaren Verhältnis von 1:3 durch den Rapid-DNA-Ligation Kit nach der Vorschrift des Herstellers ligiert. Die Transformation des Ligationsansatzes erfogte in den Stamm E. coli XL-1-blue. Positive Klone wurden durch Plasmidpräparation und Restriktionsanalyse identifiziert. Die richtige Orientierung des bioS1-Fragments in pHS1 wurde durch Restriktionsverdau und Sequenzierung bestimmt. Das erhaltene Konstrukt wurde pHS1 bioS1 (Figur 1) genannt. Die Sequenz von pHS1 bioS1 ist SEQ ID No. 5 zu entnehmen. Die abgeleitete Aminosäuresequenz von bioS1 im Vektor ist SEQ ID No. 6 zu entnehmen. 2 µg des Vektors pHS1 bioS1 wurden mit MluI verdaut und das Gen bioS1 durch ein Agarosegel isoliert. Der Vektor pHS2 wurde mit MluI verdaut mit SAP-Phospahtase dephosphorylliert und mit dem Fragment bioS1 zusammen in einer Ligation angesetzt. Der Ligationsansatz wurde in XL1-blue transformiert und positive Klone in der richtigen Orientierung durch Plasmidisolierung und Restiktionsverdau identifiziert. Der erhaltenen Vektor wurde pHS2 bioS1 (Figur 2) genannt. Die Sequenz von pHS2 bioS1 ist SEQ ID No. 9 zu entnehmen. Die abgeleitete Aminosäuresequenz von bioS1 im Vektor ist SEQ ID No. 10 zu entnehmen.

### 3. Klonierung von bioS2 (SEQ ID No. 3):

Um in E. coli weitere Gene zu klonieren, die für Genprodukte kodieren die am Aufbau von Fe-S-Clustern beteiligt sind, wurde folgender Weg beschritten:

Ein Sequenzvergleich (Programm Megalingn, Modus Clustal) von As-Sequenzen von Proteinen der Datenbank Swissprot/PIR mit einer hohen Homologie (>40%) zum NifS Protein aus A. vinelandii zeigte, daß neben dem bereits beschriebenen Sequenzmotiv I auch der N-Terminus dieser Proteine eine signifikante Konservierung zeigt. Als eine typische N-terminale konservierte Sequenz von Proteinen der NifS-Familie wurde die As-Sequenz mit der Sequenz MIYLDNXATT identifiziert und als Motiv II a bezeichnet.

Eine Analyse der Datenbank Swissprot/PIR auf eine Konservierung dieser Sequenz mit mehr als 80% zeigte ein weiteres Protein in E. coli. Von diesem Protein ist durch Edman-Abbau erhaltene N-terminale Sequenz von 11 As bekannt (Datenbankbezeichnung: UP06_Ecoli). Dieses Protein wurde als hypothetisches NifS-Homolog angesehen und in Analogie zu BioS1 als im folgenen als BioS2 bezeichnet.

Um das Gen für das Protein BioS2 zu klonieren und zu sequenzieren, wurde folgender Weg beschritten. Ausgehend von der Proteinsequenz HIU00072_10 wurde zum einen das konservierte Aminosäure-Motiv I zum anderen die oben genannte As-Sequenz von UP06_Ecoli benutzt, um degenerierte Oligonukleotide herzustellen, die in der Lage sind, ein Fragment des bioS2 Gens zu amplifizieren. Dafür wurden die beiden As-Motive HIU00072_10 (Motiv I) und UP06_Ecoli (Motiv II b, MKLPIYLDYSAT) in die korrespondierenden DNA-Sequenzen revers translatiert. Aus dem Motiv II wurde so das degenerierte Oligonukleotid PbioS2,1 (5'-ATGAARYTNCCNATHTAYYTNGAY-TAYWSNGCNAC-3') und aus dem Motiv I das degenerierte Oligonukleotid PbioS2,2 (5'-cccaghggrccrtgcagyttrtgrccrga-3') synthetisiert.

### Durchführung der PCR-Reaktion:

Als Matrize diente chromosomale DNA von E. coli W3110. Je 0,5 µg der Oligonukleotide Pbio2,1 und PbioS2,2 wurden mit je 15 pMol Nukleotidmix, 2,5 U Pwo DNA-Polymerase (Boehringer Mannheim) im Reaktionspuffer des Herstellers umgesetzt. Das Volumen der PCR-Reaktion betrug 100µl.

### Amplifikationsbedingungen:

Die Denaturierung erfolgte für 2 min bei 94 °C. Die Anlagerung der Oligonukleotide wurde bei 45 °C, die Elongation für 45 sec bei 72 °C durchgeführt. Die PCR wurde über 30 Zyklen durchgeführt. Durch die PCR konnten drei Fragmente selektiv amplifiziert werden, von denen eines die aus den Sequenzvergleichen erwartete Größe von 600 bp hatte. Dieses DNA-Fragment wurde durch Agarose-gel-Aufreinigung isoliert und mit dem Oligonukleotid PbioS2,2 sequenziert. Die erhaltene DNA-Sequenz wurde in allen sechs möglichen Leserastern translatiert. Die erhaltenen translatierten As-Sequenzen wurden dann mit der translatierten AS-Sequenz von HIU00072_10 bzw. NifS aus A. vinelandii verglichen. Eines der translatierten Leseraster zeigt eine hohe Homologie mit der As-Sequenz des beschriebenen ORF HIU00072_10 und mit bioS2 bezeichnet.

Um die gesamte DNA-Sequenz von bioS2 (= SEQ ID No. 3) zu bestimmen und zu klonieren, wurde folgender weg beschritten:

Zuerst wurde eine markierten DNA-Sonde hergestellt, die zu bioS2 homolog ist. Dies geschah mit Hilfe des PCR-DIG-Labelling-Kit (Boehringer Mannheim). Als Matrize für die Herstellung der DIG-DNA-Sonde diente das beschriebene PCR-Produkt, das durch die Oligonukleotide PbioS2,1 und PbioS2,2 hergestellt wurde.

### Die Bedingungen der PCR waren:

Einsatz: 1µl der PCR-Matrize, 5µl Nukleotid-DIG-dUTP-Mix, je 15 pMol Oligonukleotid PbioS2,1 und PbioS2,2, Puffer des Kits mit 1,75 mM MgCl₂ 0,75 µl Expand-Polymerase-Mix (Boehringer Mannheim).

### Amplifikationsbedingungen:

Aufschmelzen der DNA bei 94 °C für 2 min, Aufschmelzen der DNA 10 sec bei 94 °C, Annealing 30 sec bei 45 °C, Elongation 3.30 min bei 68 °C über 10 Zyklen, Aufschmelzen der DNA 10 sec bei 94 °C, Annealing 30 sec bei 45 °C, Elongation 3.30 min bei 68 °C, Verlängerung der Elongation für 20 sec pro Zyklus über 20 Zyklen. Aufreinigung des DIG-markierten Frgaments durch PCR-Purification Kit.

### 4. Southern-Analyse von bioS2 mit chromosomaler DNA

In weiteren Schritten wurde genomische DNA durch Restriktionsenzyme verdaut und durch Southern-Hybridisierung mit Hilfe der markierten DNA-Sonde analysiert.

Chromosomale E. coli DNA W3110 (10µg) wurde mit den folgenden Restriktionsenzymen vollständig verdaut EcoRI, BamH1, Acc65I, HindIII, SalI. Das Volumen der Ansätze betrug 50 µl, die Enzymmenge je 30 U. Die Ansätze wurden 4h inkubiert. Die derart verdaute DNA wurden durch ein 1% Agarosegel in TBE-Puffer (Sambrook, J. Fritsch, E F. Maniatis, T. 2nd ed. Cold Spring Harbor Laboratory Press., 1989, ISBN 0-87969-373-8) aufgetrennt und mit Hilfe einer Druckübertragungskammer (Stratagene) auf eine Nylon-Membran (Boehrineger Mannheim) übertragen und durch UV-Bestrahlung (Stratalinker, Stratagene) kovalent an der Membran fixiert. Die Hybridisierung erfolgte mit der DIG-markierten DNA-Sonde in DIG-Easyhyb Puffer (Boehringer Mannheim) bei 65 °C für 15 h. Die Entwicklung des Blots nach Anweisungen des Hersteller zeigt Hybridisierung der BioS2-DIG-DNA-Sonde mit Banden, die mit Größen von ca. 3 - 4 kb im Fall von Acc65I, EcoRI und HindII bestimmt wurden. BamH1 verdaute DNA zeigte eine Hybridisierung mit der bioS2-Sonde mit einem Fragment eines wesentlich höheren Molekulargewichts. Zur weiteren Klonierung wurden Fragmente von 3 - 4 kb bevorzugt weiter bearbeitet.

### Klonierung von bioS2 durch inverse PCR

Im Fall des EcoRI-Verdaus wurde ein Fragment von ca. 4 kb identifiziert, das das gesuchte Gen trägt. Das gesamte Gen wurde dann durch die Technik der inversen PCR amplifiziert und kloniert. Im ersten Schritt der inversen PCR wurde chromosomale E. coli DNA durch EcoRI vollständig verdaut. Im zweiten Schritt wurde dann die EcoRI verdaute DNA bei geringen DNA-Konzentrationen (ca.20 ng/ml) aus dem vorher beschriebenen Restriktionsverdau durch Ligase kovalent ligiert, unter Bedingungen unter denen statistisch gesehen eine intramolekulare Verknüpfung erfolgt. Im dritten Schritt wurde dann eine PCR-Reaktion durchgeführt, bei der Oligonukleotide eingesetzt werden, deren Sequenz spezifisch für das gesuchte Zielgen ist.

Spezifisch amplifizierte DNA-Segemente können anhand ihrer Größe identifiziert werden, die sich aus der Größe des Restriktionsfragments aus der Southern-Analyse und aus der Lokalisierung der Oligonukleotide im bekannten Abschnitt des Gens ergibt. Derart identifizierte Fragmente werden dann in einen geeigneten Vektor wie pBS SK Blueskript/ pCR Script (Stratagene) kloniert und sequenziert.

### Experimentelle Durchführung:

1µg chromosomale DNA aus dem Stamm W3110 wurde durch 15 U EcoRI (Boehringer Mannheim) in einem Volumen von 50 µl vollständig verdaut. Die Vollständigkeit des Verdaus wurde durch Auftrag von 30 µl auf ein Agarosegel überprüft. Fragmente dieses Verdaus chromosomaler DNA (10 µl des Verdaus = 200 ng) wurden in einem Volumen von 100 µl zusammen mit 10 µl Ligationspuffer und 2 U T4-Ligase (Boehringer Mannheim) für 15h bei 15 °C inkubiert. (intramolekulare Ligationsreaktion). Nach der Ligationsreaktion wurde die T4-Ligase durch Inkubation bei 65 °C für 20 min. inaktiviert. Von diesem Ligationsansatz wurden 5 µl als Matrize für eine PCR-Reaktion eingesetzt. Die Primer PbioS2,3 (5'-GCGTGGGTAAACTGCCTATCGACCTGAGCC-3') und PbioS2,4 (5'-CTACGCTT-CCTTCAGCCTGCCAGCCGAAA-3') wurden ausgehend von der Sequenz von bioS2 synthetisiert.

Oligonukleotid PbioS2,3 hybridisiert auf der 5'-Seite von bioS2 und bewirkt das die Elongation der Amplifikation der kodierenden Sequenz auf der 5'-Seite auf dem Gegenstrang in 3'-Richtung stattfindet.

Oligonukleotid PbioS2,4 hybridisiert auf der 3'-Seite von bioS2 und bewirkt das die Elongation der Amplifikation der kodierenden Sequenz auf der 3'-Seite auf dem kodierenden Strang in 3'-Richtung stattfindet.

Einsatz: 5µl des Ligationsansatz, 1,75 µl Desoxynukleotid-Mischung (350 µmol, Boehringer Mannheim), je 15 pMol Oligonukleotid PbioS2,5 und PbioS2,6, Puffer 1des Kits mit 1,75 mM MgCl₂ 0,75 µl Expand-Polymerase-Mix.

Bedingungen für die Amplifikation der ligierten E. coli DNA mit Primer PbioS2,3/ PbioS2,4. Expand-Kit (Boehringer Mannheim) Aufschmelzen der DNA 2 min bei 94 °C, Aufschmelzen der DNA 10 sec bei 94 °C, Annealing 30 sec bei 61 °C, Elongation 3.30 min bei 68 °C über 10 Zyklen, Aufschmelzen der DNA 10 sec bei 94 °C, Annealing 30 sec bei 61 °C, Elongation 3.30 min bei 68 °C, Verlängerung der Elongation für 20 sec pro Zyklus über 20 Zyklen.

Durch die beschriebene Amplifikation wurde ein PCR-Produkt von ca. 3 kb erhalten. Dieses DNA-Fragment zeigte in einer Southern-Hybridisierung unter stringenten Bedingungen eine deutliche Hybridisierung mit der bereits beschriebenen bioS2-DIG-DNA-Sonde.

Es wurde angenommen, daß dieses DNA-Fragment DNA-Sequenzen enthält, die zu bioS2 hochgradig homolog sind. Daher wurde dieses DNA-Fragment in einen Vektor kloniert, um es weiter zu charakterisieren und zu sequenzieren. Das DNA-Fragment wurde mit dem pCR-Skript Kit (Stratagene) zuerst laut Anweisung des Herstellers mit Pfu-Polymerase behandelt und dann in den Vektor pCR Skript ligiert. Der Ligationsansatz wurde in XL-1-blue Zellen (Stratagene) transformiert und auf LB-Amp ausplattiert. Ein positiver Klon, der ein Fragment trug, wurde durch Minipräparationsanalyse identifiziert. Die Sequenzierung ergab die in SEQ ID No. 3 wiedergegebene Gesamtsequenz (= bioS2).

BioS2 wurde dann als Expressionskassette analog zu bioS1 amplifiziert und kloniert. Dafür wurden dem Gen durch PCR mit den Oligonukleotiden PbioS2,5 (5'-CATGACGCGTAAAGAGGAGAAATTAACTATGAAATTAC-CGATTTATTTGG-3') und PbioS2,6 (5'-GCGACGCGTGATTAATGATGAGCCCAT-3') eine Erkennungstelle für MluI und eine optimierte Shine-Dalgarno-Sequenz auf der 5'Seite und eine Erkennungstelle für MluI auf der 3'Seite zugefügt.

### Durchführung der PCR:

Als Matrize wurden 0,5 µg chromosomale DNA von W3110 eingesetzt. Die Oligonukleotide PbioS2,5 und PbioS2,6 wurden in einer Konzentration von je 15 pMol eingesetzt. Die Konzentration an dNTP's betrug 200 µM. Als Polymerase wurden 2,5 U Pwo DNA-Polymerase (Boehringer Mannheim) im Reaktionspuffer des Herstellers eingesetzt. Das Volumen der PCR-Reaktion betrug 100 µl.

### Amplifikationsbedingungen:

Denaturierung 2 sec bei 94 °C, Anlagern der Oligonukleotide für 30 sec bei 55 °C, Elongation für 45 sec bei 72 °C. Die PCR wurde über 30 Zyklen durchgeführt.

Das erhaltene DNA-Produkt der richtigen Größe von ca. 1200 bp wurde durch den PCR-Purification-Kit (Boehringer Mannheim) aufgereinigt und durch MluI im geeigneten Puffer verdaut.

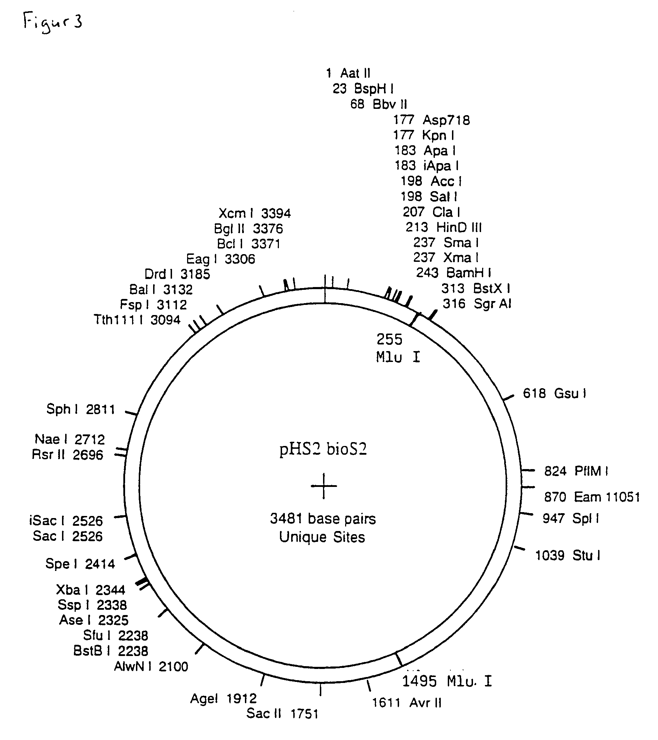

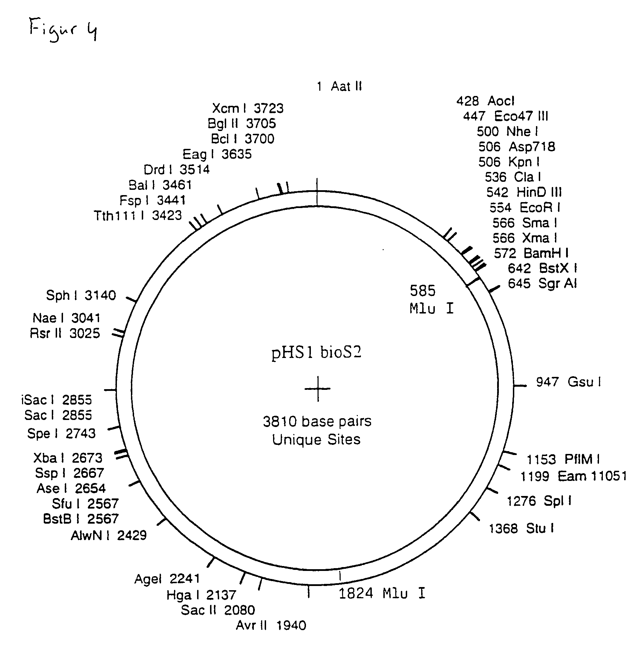

5 µg des Vektors pHS2 wurden duch MluI verdaut und durch Shrimp Alkalische Phosphatase (SAP) (Boehringer Mannheim) dephosphorylliert. Nach Denaturierung der SAP wurden Vektor und Fragment in einem molaren Verhältnis von 1:3 durch den Rapid-DNA-Ligation Kit nach der Vorschrift des Herstellers ligiert. Die Transformation des Ligationsansatzes erfolgte in den Stamm XL-1-blue. Positive Klone wurden durch Plasmidpräparation und Restriktionsanalyse identifiziert. Die richtige Orientierung des bioS2-Fragments in pHS2 wurde durch Restriktionsverdau und Sequenzierung bestimmt. Der Vektor wurde mit pHS2 bioS2 (Figur 3) bezeichnet. Die Sequenz von pHS2 bioS2 ist SEQ ID No. 11 zu entnehmen. Die abgeleitete Aminosäuresequenz von bioS2 im Vektor ist SEQ ID No. 12 zu entnehmen. Analog wurde die Klonierung von bioS2 in den Vektor pHS1 vorgenommen. Die Sequenz von pHS1 bioS2 ist SEQ ID No. 7 zu entnehmen(Figur 4). Die abgeleitete Aminosäuresequenz von bioS2 im Vektor ist SEQ ID No. 8 zu entnehmen.

### 5. Konstruktion des Plasmids pHBbio14

Die in vivo-Klonierung des Bio-Operons wurde durch einen transduzierenden Lambda-Phagen durchgeführt. Die Selektion von Lambda bio⁺ Phagen erfolgte durch die Transduktion eines E. coli bionegativen Stammes zu bio⁺. Der isolierte bio⁺ transduzierende Lambda Phage wurde propagiert und die Lambda DNA aufgereinigt. Es folgte die Excision eines 8,7 kb EcoRI/HinDIII-Fragments mit dem gesamten Biotin-Operon aus der Lambda-Phagen DNA und Ligation des Fragments in pBR322, der EcoRI/HinDIII geschnitten worden war. Positive Klone wurden durch Plasmidpräparation und Restriktionsanalyse identifiziert.

### Deletion eines 1,2 Kb Fragments 3' von bioD

Nicht benötigte Gensequenzen auf der 3'-Seite des bioD Gens wurden entfernt. Dafür wurde durch PCR-Reaktion hinter dem Stopkodon von bioD eine EcoRI-Schnittstelle eingebaut Die Entwicklung der Oligonukleotide Pbiol,1 (5'-AATAAGGAATTCTTATGTACTTTCCGGTTGCCG-3') und Pbiol,2 (5'-AACAGCAGCCTGCAGCTGGATTA-3') für diese PCR-Reaktion erfolgte nach der Operon-Sequenz von Otsuka et al. (J. Biol. Chem. 263, 1988: 19577-85).

### PCR-Bedingungen:

2,5 U Taq-Polymerase (Perkin Elmer) und je 15 pmol Primer wurden in einem Volumen von 100 µl umgesetzt. Das Annealing wurde bei 50 °C und die Elongation für 1 min bei 72 °C über 30 Zyklen durchgeführt. Isolierung und Aufreinigung eines 488 bp-Fragments erfolgte über Agarose-Gel. Verdau des erhaltenen Fragments mit EcoRI/PstI. Verdau von pHBbio1 mit EcoRI/PstI. Isolierung eines 9,5 kb Fragments.

Das 9,5 kb Fragments wurde mit dem 488 bp Fragments legiert und in XL1-blue-Zellen transformiert. Erhaltene Klone wurden durch Plasmidpräparation und durch Restriktionsanalyse der Plasmid-DNA mit den Enzymen EcoRI und HinDIII analysiert, positive Klone, die ein 5,9 kb Fragment trugen, wurden identifiziert. Es wurde ein Klon, der mit pHBbio2 bezeichnet wurde, isoliert. Von diesem Klön wurde Plasmid-DNA gewonnen. 5 µg pHBbio2 wurden mit EcoRI/HinDIII verdaut und das 5,9 kb Fragment isoliert, das die gesamten Biotin-Biosynthesegene enthielt.

5 µg des Plasmids pAT153 wurden mit EcoRI und HinDIII verdaut. Das erhaltenen 5,9 kb Fragment mit den Biotin-Biosynthesegenen wurde mit dem verdauten Vektor pAT153 ligiert und in XL-1-blue transformiert. Erhaltene Klone wurden durch Plasmidpräparation und durch Restriktionsanalyse der Plasmid-DNA mit den Enzymen EcoRI und HinDIII analysiert. Positive Klone wurden identifiziert und ein Klon mit der Bezeichnung pHbio14 isoliert.

### 6. Erhöhung der Biotin-Produktivität durch Überexpression von bioS1

Stamm BM4092 (Barker und Campbell) wurde durch eine P1-Transduktion mit Hilfe eines P1-Lysats, das auf einem recA::Tn10 tragendem Stamm gewachsen war, nach recA⁻ transduziert. Der Erfolg der Transduktion wurde durch eine erhöhte UV-Sensitivität der positiven Transduktanden nachgewiesen. Daraufhin wurde der erhaltene Stamm LU8091 mit dem Plasmid pHBbio14 nach der CaCl₂-Methode transformiert und auf LB-Ampizillin 100 µg/ml angezogen. Ein Klon wurde isoliert, und dieser wurde jeweils mit den Plasmiden pHS1 bioS1 und pHS2 bioS2 durch die CaCl₂-Methode transformiert und auf LB-Agar, Ampizillin 100 µg/ml und Kanamyzin 25 µg/ml selektioniert.

Je eine Kolonie der jeweiligen Transformanden wurde in einer DYT-Kultur mit dem entsprechendem Antibiotika angeimpft und für 12 h inkubiert. Die Übernachtkultur (= ÜNK) wurde eingesetzt um eine 10 ml Kultur in TB-Medium (Sambrook, J. Fritsch, E F. Maniatis, T. 2nd ed. Cold Spring Harbor Laboratory Press., 1989 ISBN 0-87969-373-8) mit den entsprechenden Antibiotika anzuimpfen und über 24h Stunden angezogen. Nach Ende des Wachstums wurden die Zellen vom Kulturüberstand durch Zentrifugation abgetrennt und die Biotin- und Dethiobiotin-Konzentration durch einen ELISA mit Streptavidin und Avidin im Überstand bestimmt. Die Ergebnisse dieser Bestimmung sind Tabelle I zu entnehmen.

**Tabelle I: Bestimmung der Biotin- und Dethiobiotinkonzentration**

| Stamm | Plasmid I | Plasmid II | Biotin mg/l | Dethiobiotin mg/l |
|---|---|---|---|---|
| Lu8091 | pHBbio14 | | 9,4 | 45,6 |
| Lu8091 | pHBbio14 | pHS1 bioS1 | 15,3 | 19,7 |
| Lu8091 | pHBbio14 | pHS2 bioS1 | 19,2 | 15,8 |

## Patentansprüche

1. Verfahren zur Herstellung von Biotin, **dadurch gekennzeichnet, dass** man ein Biotingen mit der Sequenz SEQ ID No. 1 oder SEQ ID No. 3 in einem prokaryontischen oder eukaryontischen Wirtsorganismus, der in der Lage ist Biotin zu synthetisieren, exprimiert, diesen züchtet und das synthetisierte Biotin direkt, nach Abtrennung der Biomasse oder nach Reinigung des Biotins verwendet.

2. Verfahren nach Anspruch 1, **dadurch gekennzeichnet, dass** die Expression der Biotingene nach Anspruch 1 zu einer gesteigerten Umwandlung von Dethiobiotin in Biotin führt.

3. Verfahren nach den Anprüchen 1 bis 2, **dadurch gekennzeichnet, dass** als Wirtsorganismus ein Organismus ausgewählt aus der Gruppe der Gattungen Escherichia, Citrobacter, Serratia, Klebsiella, Salmonella, Pseudomonas, Comamonas, Acinetobacter, Azotobacter, Chromobacterium, Bacillus, Chlostridium, Arthrobacter, Corynebacterium, Brevibacterium, Lactococcus, Lactobacillus, Streptomyces, Rhizobium, Agrobacterium, Staphylococcus, Rhodotorula, Sprorobolomyces, Yarrowia, Schizosaccharomyces oder Saccharomyces verwendet wird.

4. Verfahren nach den Ansprüchen 1 bis 3, **dadurch gekennzeichnet, dass** als Wirtsorganismus eine regulationsdefekte Biotinmutante verwendet wird.

5. Verfahren nach den Ansprüchen 1 bis 4, **dadurch gekennzeichnet, dass** die Expression mindestens einer Kopie der Biotingene nach Anspruch 1 alleine oder mit einer oder mehreren Kopien mindestens eines weiteren Biotingens ausgewählt aus der Gruppe bioA, bioB, bioF, bioC, bioD, bioH, bioP, bioW, bioX, bioY oder bioR in einem prokaryontischen oder eukaryontischen Wirtsorganismus erfolgt.

6. Genkonstrukt enthaltend ein Biotingen mit der SEQ ID No. 1 oder SEQ ID No. 3, das mit einem oder mehreren Regulationssignalen zur Erhöhung der Genexpression und/oder Proteinexpression funktionell verknüpft ist und/oder dessen natürliche Regulation ausgeschaltet wurde, zusammen mit einer oder mehreren Kopien mindestens eines weiteren Gens ausgewählt aus der Gruppe bioA, bioB, bioF, bioC, bioD, bioH, bioP, bioW, bioX, bioY oder bioR, ausgenommen Genkonstrukte, die ein nifS-Gen aus Klebsiella pneumoniae und ein bioB-Gen aus Escherichia coli enthalten.

7. Organismen enthaltend ein Genkonstrukt gemäß Anpruch 6.

8. Verwendung eines Genkonstrukts gemäß Anpruch 6 zur Herstellung von Biotin.

## Claims

1. A process for preparing biotin, which comprises expressing a biotin gene having the sequence SEQ ID No. 1 or SEQ ID No. 3, in a prokaryotic or eukaryotic host organism able to synthesize biotin, cultivating this organism and using the synthesized biotin directly, after removal of the biomass or after purification of the biotin.

2. The process according to claim 1, wherein the expression of the biotin genes as set forth in claim 1 leads to an increased conversion of dethiobiotin into biotin.

3. The process according to claim 1 to 2, wherein the host organism used is an organism selected from the group of genera Escherichia, Citrobacter, Serratia, Klebsiella, Salmonella, Pseudomonas, Comamonas, Acinetobacter, Azotobacter, Chromobacterium, Bacillus, Clostridium, Arthrobacter, Corynebacterium, Brevibacterium, Lactococcus, Lactobacillus, Streptomyces, Rhizobium, Agrobacterium, Staphylococcus, Rhodotorula, Sporobolomyces, Yarrowia, Schizosaccharomyces or Saccharomyces.

4. The process according to any of claims 1 to 3, wherein a regulation-defective biotin mutant is used as host organism.

5. The process according to any of claims 1 to 4, wherein there is expression of at least one copy of the biotin genes as set forth in claim 1, alone or with one or more copies of at least one other biotin gene selected from the group of bioA, bioB, bioF, bioC, bioD, bioH, bioP, bioW, bioX, bioY or bioR in a prokaryotic or eukaryotic host organism.

6. A gene construct comprising a biotin gene having SEQ ID No. 1 or SEQ ID No. 3, which is functionally linked to one or more regulatory signals to increase gene expression and/or protein expression, and/or whose natural regulation has been switched off, together with one or more copies of at least one other gene selected from the group of bioA, bioB, bioF, bioC, bioD, bioH, bioP, bioW, bioX, bioY or bioR in the vector, with the exception of gene constructs which comprise a nifS gene from Klebsiella pneumoniae a bioB gene from Escherichia coli.

7. An organism comprising a gene construct according to claim 6.

8. The use of a gene construct according to claim 6 for preparing biotin.

## Revendications

1. Procédé de fabrication de biotine, **caractérisé en ce que** l'on exprime un gène de biotine, dont la séquence est représentée par la SEQ ID n° 1 ou la SEQ ID n° 3, dans un organisme hôte procaryote ou eucaryote qui est en mesure de synthétiser la biotine, **en ce que** l'on cultive ce dernier et **en ce que** l'on utilise la biotine synthétisée directement, ou après l'avoir séparée de la biomasse ou après l'avoir purifiée.

2. Procédé selon la revendication 1, **caractérisé en ce que** l'expression des gènes de biotine selon la revendication 1 conduit à une conversion renforcée de la déthiobiotine en biotine.

3. Procédé selon les revendications 1 à 2, **caractérisé en ce que** l'on utilise, comme organisme hôte, un organisme choisi dans le groupe des souches Escherichia, Citrobacter, Serratia, Klebsiella, Salmonella, Pseudomonas, Comamonas, Acinetobacter, Azotobacter, Chromobacterium, Bacillus, Chlostridium, Arthrobacter, Corynebacterium, Brevibacterium, Lactococcus, Lactobacillus, Streptomyces, Rhizobium, Agrobacterium, Staphylococcus, Rhodotorula, Sprorobolomyces, Yarrowia, Schizosaccharomyces ou Saccharomyces.

4. Procédé selon les revendications 1 à 3, **caractérisé en ce que** l'on utilise, comme organisme hôte, un mutant de biotine présentant une anomalie en matière de régulation.

5. Procédé selon les revendications 1 à 4, **caractérisé en ce que** l'expression d'au moins une copie des gènes de biotine selon la revendication 1 s'effectue seule ou avec une ou plusieurs copies d'au moins un autre gène de biotine, choisi dans le groupe constitué des gènes bioA, bioB, bioF, bioC, bioD, bioH, bioP, bioW, bioX, bioY ou bioR, dans un organisme hôte procaryote ou eucaryote.

6. Construction génique contenant un gène de biotine ayant la SEQ ID n° 1 ou la SEQ ID n° 3, qui est relié de manière fonctionnelle à un ou plusieurs signaux de régulation visant à augmenter l'expression génique et/ou l'expression protéique et/ou dont la régulation naturelle a été éteinte, conjointement avec une ou plusieurs copies d'au moins un autre gène, choisi dans le groupe constitué des gènes bioA, bioB, bioF, bioC, bioD, bioH, bioP, bioW, bioX, bioY ou bioR, à l'exception des constructions géniques qui contiennent un gène nifS de la souche Klebsiella pneumoniae et un gène bioB de la souche Escherichia coli.

7. Organismes contenant une construction génique selon la revendication 6.

8. Utilisation d'une construction génique selon la revendication 6 pour la production de biotine.
